# EUROPÄISCHE PATENTSCHRIFT

(11) **EP 1 570 065 B1**
(45) Veröffentlichungstag und Bekanntmachung des Hinweises auf die Patenterteilung: **17.10.2007**
(21) Anmeldenummer: 03780119.8
(22) Anmeldetag: 04.12.2003
(51) Int. Cl.: C12P 7/00, C09D 157/06

(54) **ENZYMATISCHE HERSTELLUNG URETHANGRUPPENHALTIGER (METH)ACRYLSÄUREEESTER**
ENZYMATIC PRODUCTION OF (METH)ACRYLIC ESTERS THAT CONTAIN URETHANE GROUPS
PRODUCTION ENZYMATIQUE D'ESTERS DE L'ACIDE (METH)ACRYLIQUE COMPORTANT DES GROUPES URETHANE

(30) Priorität: 05.12.2002 DE 10257094
(43) Veröffentlichungstag der Anmeldung: 07.09.2005
(73) Patentinhaber: BASF Aktiengesellschaft, 67056 Ludwigshafen (DE)
(72) Erfinder: DIETSCHE, Frank, 69198 Schriesheim (DE); HÄRING, Dietmar, 69198 Schriesheim (DE); WAGNER, Eva, 67346 Speyer (DE); SCHWALM, Reinhold, 67157 Wachenheim (DE); RINK, Heinz-Peter, 48153 Münster (DE); BECK, Erich, 68526 Ladenburg (DE)
(86) Internationale Anmeldenummer: PCT/EP2003/013689
(87) Internationale Veröffentlichungsnummer: WO 2004/050888

(56) Entgegenhaltungen:
- EP-A- 0 999 229
- EP-A- 0 999 230
- EP-A- 1 043 351
- EP-A- 1 162 218
- WO-A-98/50345
- DE-A- 2 027 465
- US-A- 3 718 692
- DERANGO REGINA ET AL: "The lipase-catalyzed synthesis of carbamoyloxyethyl methacrylate" BIOTECHNOLOGY LETTERS, Bd. 16, Nr. 3, 1994, Seiten 241-246, XP009026932 ISSN: 0141-5492 in der Anmeldung erwähnt
- PATENT ABSTRACTS OF JAPAN vol. 2000, no. 19, 5. Juni 2001 (2001-06-05) & JP 2001 040039 A (DAINIPPON INK & CHEM INC), 13. Februar 2001 (2001-02-13) in der Anmeldung erwähnt
- PROBST & KOLB: "Homo- und Copolymerisation von N,N-disubstituierten Carbamoyloxyalkylacrylaten und -methacyrlaten." MAROMOLECULAR CHEMISTRY, Bd. 177, - 1976 Seiten 2681-2695, XP009026952

## Beschreibung

Die vorliegende Erfindung betrifft ein Verfahren zur Herstellung von urethangruppenhaltigen (Meth)acrylsäureestern und deren Verwendung in strahlungshärtbaren Beschichtungsmassen.

Die Herstellung von (Meth)acrylsäureestern erfolgt zumeist durch säure- oder basenkatalysierte Ver- oder Umesterung von (Meth)acrylsäure oder anderen (Meth)acryläureestern mit Alkoholen bei Temperaturen von 40 bis deutlich über 100°C. Aufgrund der hohen Temperaturen ist der Zusatz hoher Mengen von Polymerisationsinhibitoren erforderlich, um eine unerwünschte Polymerisierung der Monomeren zu unterdrücken. Dabei entstehen oft komplexe und bisweilen gefärbte Produktgemische. Zur Entfernung von Färbungen und unumgesetzter Reaktanden werden die Produktgemische durch aufwendige alkalische Wäschen aufgearbeitet. Das Waschverfahren ist langwierig und kostspielig, da sich vor allem teilveresterte Produkte nur langsam extrahieren und abtrennen lassen.

Die Herstellung urethangruppenhaltiger (Meth)acryate über eine konventionelle sauerkatalysierte Veresterung ist zudem schwierig, da Urethangruppen säureempfindlich sind.

JP-A 2001-40039 beschreibt carbamatgruppenhaltige (Meth)acrylsäureester, die über eine sauerkatalysierte Veresterung hergestellt werden.

Nachteilig an dem beschriebenen Verfahren ist, daß die Reinheit des erhaltenen Produkts lediglich 75,9 % bei einer Massenbilanz von 95 % beträgt.

EP-A1 36 813 beschreibt die zweistufige Herstellung N-substituierter, carbamatgruppenhaltiger Acrylate durch Umsetzung von mehrfach hydroxyalkylierten Acrylaten mit Isocyanaten.

Nachteilig an dem beschriebenen Verfahren ist die Beschränkung auf solche Substrate, die als Isocyanate verfügbar sind. So sind beispielsweise N,N-disubstituierte Carbamate nach diesem Verfahren nicht herstellbar, ebenfalls solche mit N-Substituenten, die gegenüber Isocyanat reaktive Gruppen tragen. Für die Umsetzung mit dem Isocyanat sind zudem toxische Zinnverbindungen als Katalysator notwendig.

Die Herstellung von (Meth)acrylsäureestern durch eine enzymatische Ver- oder Umsterung ist bekannt.

Hajjar et al. beschreiben in *Biotechnol. Lett.* **1990**, *12*, 825-830 die enzymatische Umsterung von cyclischen und offenkettigen Alkandiolen mit Ethylacrylat mit einer Lipase aus *Chromobacterium viscosum.* Die Reaktionen laufen bei einem 18-fachen molaren Überschuß des Alkylacrylats gegenüber dem Diol in einem lösungsmittelfreien System ab. Es entstehen Mischungen aus Mono- und Diacrylaten.

US 5240835 beschreibt die Umesterung von Alkylacrylaten mit Alkoholen unter Katalyse eines Biokatalysators aus *Corynebacterium oxydans.* Beispielhaft wird dort die Reaktion von einem 96-fachen molaren Überschuß Ethylacrylat mit 2,2-Dimethyl-1,3-proandiol aufgeführt. Lediglich 21 % Ausbeute wurden nach 3 Tagen bei 30 °C erhalten.

Derango et al. beschrieben in Biotechnol. Lett. 1994, 16, 241-246 die Lipase-katalysierte Herstellung von Carbamoyloxyethylmethacrylat durch Umesterung von 2-Hydroxyethylcarbamat mit Vinylmethacrylat. Eine vollständige Umsetzung wird erreicht durch das spezielle Edukt Vinylmethacrylat, da freigesetzter Vinylalkohol dem Reaktionsgleichgewicht als Acetaldehyd entzogen wird. Nachteilig an diesem Verfahren ist, daß Vinylmethacrylat nicht kommerziell verfügbar ist.

Die Verwendung von urethangruppenhaltigen (Meth)acrylsäureestern in strahlungshärtbaren Beschichtungsmassen ist aus EP-A1 263 749 bekannt.

Aufgabe der vorliegenden Erfindung war es, ein Verfahren zur Verfügung zu stellen, mit dem urethangruppenhaltige (Meth)acrylsäureestern in hohen Umsätzen und hohen Reinheiten aus einfachen, toxikologisch unbedenklicher als Vinylmethacrylat eingeschätzten Edukten herstellbar sind. Die Synthese sollte unter milden Bedingungen ablaufen, so daß Produkte mit einer niedrigen Farbzahl und Viskosität resultieren.

Die Aufgabe wurde gelöst durch ein Verfahren zur Herstellung urethangruppenhaltiger (Meth)acrylsäureester (F) durch
c) Umsetzung eines urethangruppenhaltigen Alkohols (C) mit (Meth)acrylsäure oder einem Ester von (Meth)acrylsäure mit einem gesättigten Alkohol (D) und
d) gegebenenfalls Aufreinigung des Reaktionsgemisches aus c), wobei man die Umsetzung c) in Gegenwart eines Enzyms (E) durchführt.

Mit Hilfe des erfindungsgemäßen Verfahrens ist die Herstellung urethangruppenhaltiger (Meth)acrylsäureester in hoher chemischer und Raum-Zeit-Ausbeute und unter milden Bedingungen möglich. Mit dem erfindungsgemäßen Verfahren lassen sich vorteilhafterweise weniger gefärbte und/oder weniger viskose urethangruppenhaltiger (Meth)acrylsäureester herstellen, als nach dem Stand der Technik. Als weiterer Vorteil kann der Einsatz von Polymerisationsinhibitoren verringert und besonders vorteilhaft auf diese verzichtet werden.

Urethangruppen im Sinne dieser Schrift sind O-substituierte und N-un-, mono- oder disubstituierte Strukturelemente der Formel >N-C(=O)-O-.

(Meth)acrylsäure steht in dieser Schrift für Methacrylsäure und Acrylsäure, bevorzugt für Acrylsäure.

Urethangruppenhaltige Alkohole (C) sind solche Verbindungen, die mindestens eine Urethangruppe, bevorzugt 1 bis 10, besonders bevorzugt 1 bis 5, ganz besonders bevorzugt 1 bis 2 und insbesondere eine Urethangruppe, sowie mindestens eine Hydroxygruppe (-OH), bevorzugt 1 bis 10, besonders bevorzugt 1 bis 6, ganz besonders bevorzugt 1 bis 3, insbesondere 1 bis 2 und speziell eine Hydroxygruppe enthalten.

Bevorzugte urethangruppenhaltige Alkohole (C) weisen ein durchschnittliches Molgewicht von 105 bis 800.000 g/mol auf, bevorzugt 120 bis 25.000, besonders bevorzugt 200 bis 5000 und ganz besonders bevorzugt 400 bis 4500 g/mol.

Besonders bevorzugte urethangruppenhaltige Alkohole (C) sind solche, die erhältlich sind durch
a) Umsetzung eines Amins (A) mit einem Carbonat (B) und
b) gegebenenfalls Aufreinigung des aus a) erhältlichen Reaktionsgemisches.

Amine sind dabei Ammoniak, primäre oder sekundäre Amine, Carbonate sind O,O'-disubstituierte Carbonate mit dem Strukturemelement -O-C(=O)-O-.

Ganz besonders bevorzugte urethangruppenhaltige Alkohole (C) sind solche, die erhältlich sind durch eine Umsetzung gemäß Formel 1, 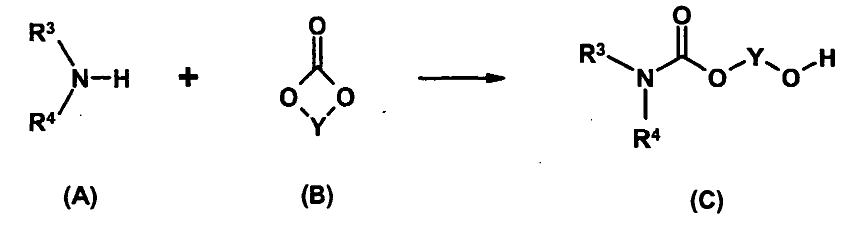 worin
- R³, R⁴: unabhängig voneinander Wasserstoff, C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ -C₁₈-Alkenyl,
C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, oder eine Gruppe der Formel -[Xᵢ]ₖ-H,
- Y: C₂-C₂₀-Alkylen, C₅-C₁₂-Cycloakylen oder durch ein- oder mehrere Sauerstoff- und/oder Schwefelatome und/oder ein oder mehrere substituierte oder unsubstituierte Iminogruppen und/oder durch eine oder mehrere Cycloalkyl-,
-(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können,
- k: für eine Zahl von 1 bis 50 und
- Xᵢ: für jedes i = 1 bis k unabhängig voneinander ausgewählt sein kann aus der Gruppe -CH₂-CH₂-O-, -CH₂-CH₂-N(H)-. -CH₂-CH₂-CH₂-N(H)-, -CH₂-CH(NH₂)-, -CH₂-CH(NHCHO)-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂- CH₂-O-, -CH₂-CHVin-O-, -CHVin-CH₂-O-, -CH₂CHPh-O- und -CHPh-CH₂O-, worin Ph für Phenyl und Vin für Vinyl steht,
bedeuten.

R³ und R⁴ können auch gemeinsam einen Ring bilden.

Bevorzugt sind R³ und R⁴ unabhängig voneinander Wasserstoff, C₁- C₁₂-Alkyl, C₅ - C₆-Cycloalkyl oder eine Gruppe der Formel -[Xᵢ]ₖ-H, besonders bevorzugt sind R³ und R⁴ unabhängig voneinander Wasserstoff, C₁ - C₄-Alkyl, C₅ - C₆-Cycloalkyl oder eine Gruppe der Formel -[Xᵢ]ₖ-H und ganz besonders bevorzugt Wasserstoff, C₁ - C₄-Alkyl, oder eine Gruppe der Formel -[Xᵢ]ₖ-H. Insbesondere ist einer der Reste R³ und R⁴ Wasserstoff und der andere C₁ - C₄-Alkyl, oder eine Gruppe der Formel -[Xᵢ]ₖ-H.

Y ist bevorzugt C₂-C₁₀-Alkylen, besonders bevorzugt C₂-C₆-Alkylen, ganz besonders bevorzugt C₂-C₄-Alkylen, insbesondere C₂-C₃-Alkylen und speziell C₂-Alkylen, wobei die genannten Reste jeweils durch Aryl, Alkyl, Aryloxy, Alkyloxy, Heteroatome und/oder Heterocyclen substituiert sein können. k ist bevorzugt 1 bis 30, besonders bevorzugt 1 bis 20, ganz besonders bevorzugt 1 bis 10 und insbesondere 1 bis 5.

Bevorzugte Xᵢ sind -CH₂-CH₂-O-, -CH2-CH₂-N(H)-, -CH₂-CH₂-CH₂-N(H)-, -CH₂-CH(NH₂)-, -CH₂-CH(NHCHO)-, -CH₂-CH(CH₃)-O- und -CH(CH₃)-CH₂O-, besonders bevorzugt ist -CH₂-CH₂-O-, -CH₂-CH₂-N(H)-, -CH₂-CH₂-CH₂-N(H)- und -CH₂-CH(NH₂)-, ganz besonders bevorzugt ist -CH₂-CH₂-O-, -CH₂-CH₂-N(H)- und -CH₂-CH₂-CH₂-N(H)-.

Beispiele für R³ und/oder R⁴ sind Wasserstoff, Methyl, Ethyl, iso-Propyl, n-Propyl, n-Butyl, *iso*-Butyl, *sek*-Butyl, *tert*-Butyl, *n*-Hexyl, *n*-Heptyl, *n*-Octyl, *n*-Decyl, *n*-Dodecyl, n-Tetradecyl, n-Hexadecyl, n-Octadecyl, n-Eicosyl, 2-Ethylhexyl, Cyclopentyl, Cyclohexyl, Cyclooctyl, Cyclododecyl, 2-Hydroxyethyl, 2-Hydroxypropyl, 1-Hydroxypropyl, 5-Hy droxy-3-oxa-pentyl, 8-Hydroxy-3,6-dioxa-octyl oder 11-Hydroxy-3,6,9-trioxa-undecyl.

Beispiele für Y sind 1,2-Ethylen, 1,2-Propylen, 1,1-Dimethyl-1,2-ethylen, 1-Hydroxy methyl-1,2-ethylen, 2-Hydroxy-1,3-propylen, 1,3-Propylen, 1,4-Butylen, 1,6-Hexylen, 2-Methyl-1,3-Propylen, 2-Ethyl-1,3-Propylen, 2,2-Dimethyl-1,3-Propylen und 2,2-Di methyl-1,4-butylen, bevorzugt sind 1,2-Ethylen, 1,2-Propylen, 1,3-Propylen, besonders bevorzugt sind 1,2-Ethylen und 1,2-Propylen und ganz besonders bevorzugt ist 1,2-Ethylen.

Beispielhafte Amine (A) sind Ammoniak, Methylamin, Dimethylamin, Ethylamin, Diethylamin, *iso*-Propylamin, Di-iso-Propylamin, n-Butylamin, Di-n-Butylamin, *tert*-Butylamin, Monoethanolamin, Diethanolamin, Propanolamin, Dipropanolamin, Piperidin, Piperazin, Pyrrolidin, Cyclopentylamin, Cyclohexylamin, Anilin, Ethylendiamin, Diethylentriamin, Triethylentetramin, Tertraethylenpentamin und Polyethylenimine mit einem gewichtsmittleren Molekulargewicht M_{w} von 200 bis 25.000 g/mol, bevorzugt von 400 bis 8.000, besonders bevorzugt von 750 bis 5000 und ganz besonders bevorzugt von 800 bis 3.000 g/mol. Erfindungsgemäß verwendbare Polyethylenimine weisen beispielsweise ein Verhältnis von primären:sekundären:tertiären Amingruppen von 1: 0,75 -1,25 : 0,4-0,8 auf. Selbstverständlich können auch höhermolekulare Polyethylenimine eingesetzt werden, beispielsweise bis zu einem Molekulargewicht M_{w} von 2.000.000, bevorzugt bis zu 750.000, da solche Polyethylenimine jedoch zumeist in wäßriger Lösung vorliegen, sollte das Lösungsmittel vor dem Einsatz in der erfindungsgemäßen Reaktion entfernt und/oder durch ein anderes ersetzt werden.

Weitere Amine (A) können beispielsweise hydrierte Polyacrylnitrile, geradkettige, verzweigte oder dendritische Polymere mit Aminofunktionen oder zumindest teilweise hydrolysierte
Poly-N-Vinylformamide sein.

Geradkettige Polymere mit Aminofunktionen sind beispielsweise Polyethylenglykole, Polypropylenglykole, gemischte Polyalkylenoxide, Poly-1,3-propandiole, Poly-THF oder alkoxlierte Polyole oder Monoole, bei denen mindestens eine endständige Hydroxygruppe durch eine Aminogruppe ersetzt worden ist, sowie aminofunktionalisierte Polyisobutene, jeweils mit einem gewichtsmittleren Molekulargewicht M_{w} von 200 bis 25.000 g/mol, bevorzugt von 400 bis 8.000, besonders bevorzugt von 750 bis 5000 und ganz besonders bevorzugt von 800 bis 3.000 g/mol. Beispiele dafür sind Jeffamine® der Firma Huntsman Corp., Houston.

Verzweigte Polymere mit Aminofunktionen sind beispielsweise beschrieben in WO 93/14147, S. 2, Z. 3 - S. 6, Z. 14, deren Herstellung ist in derselben Schrift sowie in WO 95/02008 und WO 97/23514 beschrieben, oder solche verzweigten Polymere, deren Herstellung in WO 95/20619 beschrieben ist, sowie die in Biomacrololecules, 2002, 3, 926 - 936 beschriebenen Polyethylenglykol-Polyethylenimin-Blockpolymere.

Bevorzugte verzeigte Polymere sind beispielsweise die auf 1,4-Diaminobutan gestarteten, durch abwechselnde Michael-Addition von Acrylnitril und Hydrierung der Nitrilgruppe erhältlichen Dendrimere der 1. Dendrimergeneration (Astramol® Am-4, der Firma DSM, Niederlande,
CAS-Nr. [120239-63-6]), der 2. Generation (Astramol® Am-8, der Firma DSM, Niederlande, CAS-Nr. [154487-83-9]), der 3. Generation (Astramol® Am-16,
CAS-Nr. [154487-85-1]), der 4. Generation (Astramol® Am-32, CAS-Nr. [163611-04-9]) oder der 5. Generation (Astramol® Am-64, CAS-Nr. [163611-05-0]).

Zumindest teilweise hydrolysierte Poly-N-Vinylformamide sind beispielsweise beschrieben in EP B1 71 050, S. 1, Z. 31 bis S. 4, Z. 54. Bevorzugt sind solche hydrolysierten Poly-N-Vinylformamide mit einem K-Wert (nach Fikentscher gemessen in 0,5 Gew.-%iger wäßriger Kochsalzlösung bei 25 °C)) zwischen 10 und 110, wobei K-Werte zwischen 30 und 80 besonders bevorzugt sind, und einem Spaltgrad (Hydrolysegrad der Formylgruppe) von 10 bis 100 mol%, bevorzugt 10 bis 80, besonders bevorzugt 20 bis 60 und ganz besonders bevorzugt 30 bis 50 mol%.

Beispielhafte Carbonate (B) sind Ethylencarbonat, 1,3-Propylencarbonat und 1,2-Propylencarbonat.

Die Umsetzung eines Amins (A) mit einem Carbonat (B) ist an sich bekannt, beispielsweise aus US 4,820,830, Sp. 4, Z. 44 bis Sp. 5, Z. 9, , und nicht beschränkt.

Typischerweise werden das Amin (A) und das Carbonat (B) in einer Stöchiometrie von 0,7 bis 1,2 mol Amin : 1 mol Carbonat, bevorzugt 0,8 -1,2 : 1, besonders bevorzugt 0,9 -1,1:1, ganz besonders bevorzugt 0,95 -1,1:1 und insbesondere 1:1 mol/mol miteinander umgesetzt.
Die Umsetzung erfolgt in der Regel bei einer Temperatur von 0 bis 120 °C, besonders bei 20 bis 100, ganz besonders bevorzugt 30 bis 80 und ganz besonders bevorzugt 40 bis 80 °C.

Die Umsetzung ist in der Regel innerhalb von 12 Stunden beendet, bevorzugt innerhalb von 15 Minuten bis 10 Stunden, besonders bevorzugt in 30 Minuten bis 8 Stunden, ganz besonders bevorzugt 45 Minuten bis 6 Stunden und insbesondere innerhalb von 1 bis 4 Stunden.

Die Gesamtaminzahl gem. DIN 53176 des Reaktionsproduktes sollte nicht mehr als 200 mg KOH/g betragen, vorzugsweise nicht mehr als 100 und ganz besonders bevorzugt nicht mehr als 80 mg KOH/g.

Die Umsetzung kann ohne Lösungsmittel durchgeführt werden oder in Anwesenheit eines solchen, beispielsweise Alkohole, Ether, Ketone, Kohlenwasserstoffe oder Wasser, bevorzugt ohne Lösungsmittel.

Das aus a) erhältliche Reaktionsgemisches kann in einem weiteren Schritt b) falls gewünscht aufgereinigt werden, beispielsweise durch Filtration, Destillation, Rektifikation, Chromatographie, Behandlung mit Ionentauschern, Adsorbentien, neutraler, saurer und/oder alkalischer Wäsche, Strippen oder Kristallisation.

Eine bevorzugte Ausführungsform der vorliegenden Erfindung stellen urethangruppenhaltige (Meth)acrylsäureester dar, erhältlich durch
a) Umsetzung eines Polyethylenimins, eines hydrierten Polyacrylnitrils, eines verzweigten Polymers mit Aminofunktionen oder eines zumindest teilweise hydrolysierten Poly-N-Vinylformamids mit einem gewichts mittleren Molekulargewicht M_{w} von 200 bis 1.000.000, bevorzugt 200 - 750.000, besonders bevorzugt 200 - 25.000, ganz besonders bevorzugt 400 - 8.000, insbesondere 750 - 5.000 und speziell 800 - 3.000 g/mol mit einem Carbonat (B) bei einer Temperatur von 0 bis 120 °C,
b) gegebenenfalls Aufreinigung des aus a) erhältlichen Reaktionsgemisches,
c) Umsetzung des Reaktionsgemischs aus a) oder b) mit (Meth)acrylsäure oder einem Ester von (Meth)acrylsäure mit einem gesättigten Alkohol (D) in Gegenwart eines Enzyms (E) und
d) gegebenenfalls Aufreinigung des Reaktionsgemisches aus c).

Bevorzugt ist die Umsetzung von linearen oder verzweigten Polyethyleniminen, Dendrimeren mit Aminofunktionen oder zumindest teilweise hydrolysierten Poly-N-Vinylformamiden, besonders bevorzugt von Polyethyleniminen oder Dendrimeren mit Aminofunktionen und ganz besonders bevorzugt von Polyethyleniminen.

In Schritt c) erfolgt die Um- oder Veresterung des urethangruppenhaltigen Alkohols mit mindestens einem (Meth)acrylat oder (Meth)acrylsäure (D) in Anwesenheit eines die Um- oder Veres-terung katalysierenden Enzyms (E).

Verbindungen (D) können (Meth)acrylsäure oder Ester von (Meth)acrylsäure mit einem gesättigten Alkohol sein, bevorzugt (Meth)acrylsäure und deren gesättigten C₁ - C₁₀-Alkylester.

Gesättigt bedeutet im Rahmen dieser Schrift Verbindungen ohne C-C-Mehrfachbindungen (außer selbstverständlich die C=C-Doppelbindung in den (Meth)acryleinheiten).

Beispiele für Verbindungen (D) sind (Meth)acrylsäure und (Meth)acrylsäuremethyl-, -ethyl-, -n-butyl-, -iso-butyl-, n-octyl- und -2-Ethylhexylester, 1,2-Ethylenglycoldi- und -mono(meth)acrylat, 1,4-Butandioldi- und -mono(meth)acrylat, 1,6-Hexandioldi- und -mono(meth)acrylat, Trimethylolpropantri(meth)acrylat und Pentaerythrittetra(meth)-acrylat.

Besonders bevorzugt sind (Meth)acrylsäure und (Meth)acrylsäuremethyl-, -ethyl-, -n-butyl- und
-2-Ethylhexylester und ganz besonders bevorzugt (Meth)acrylsäuremethyl-, -ethyl- und -n-butylester.

Die enzymatische Um- oder Veresterung mit einem (Meth)acrylat oder (Meth)acrylsäure erfolgt im Allgemeinen bei 0 bis 100°C, bevorzugt 20 bis 80 °C, besonders bevorzugt 20 bis 70°C, ganz besonders bevorzugt 20 bis 60 °C.

Erfindungsgemäß einsetzbare Enzyme (E) sind beispielsweise ausgewählt unter Hydrolasen, Esterasen (E.C. 3.1.-.-), Lipasen (E.C. 3.1.1.3), Glykosylasen (E.C. 3.2.-.-) und Proteasen (E.C. 3.4.-.-) in freier oder auf einem Träger chemisch oder physikalisch immobilisierter Form, bevorzugt Lipasen, Esterasen oder Proteasen. Besonders bevorzugt sind Novozyme 435 (Lipase aus *Candida antartica* B) oder Lipase aus Aspergillus sp., Aspergillus niger sp., Mucor sp., Penicilium cyclopium sp., Geotricum candidum sp., Rhizopus javanicus, Burholderia sp., Candida sp., Pseudomonas sp., oder Schweinepankreas, ganz besonders bevorzugt sind Lipase aus *Candida antartica* B oder aus Burholderia sp.

Der Enzymgehalt im Reaktionsmedium liegt in der Regel im Bereich von etwa 0,1 bis 10 Gew.%, bezogen auf die Summe der eingesetzten Komponenten (C) und (D). Die Reaktionszeit hängt unter anderem von der Temperatur, der verwendeten Menge und der Aktivität des Enzymkatalysators und vom geforderten Umsatz ab sowie vom urethangruppenhaltigen Alkohol. Bevorzugt wird die Reaktionszeit so angepaßt, daß der Umsatz aller ursprünglich im Alkohol (C) enthaltenden Hydroxyfunktionen mindestens 70%, bevorzugt mindestens 80, besonders bevorzugt mindestens 90 und ganz besonders bevorzugt mindestens 95 % beträgt. In der Regel sind dafür 1 bis 48 Stunden und bevorzugt 1 bis 12 Stunden ausreichend.

Das molare Verhältnis von (Meth)acrylsäureverbindung (D) (bezogen auf die (Meth)-acryleinheiten) zu urethangruppenhaltigem Alkohol (C) (bezogen auf Hydroxygruppen) kann in einem weiten Bereich, wie z.B. im Verhältnis 100:1 bis 1:1, bevorzugt 50:1 bis 1: 1, besonders bevorzugt 20:1 bis 1:1 und ganz besonders bevorzugt 10:1 bis 1:1, schwanken.

Die Reaktion kann in organischen Lösungsmitteln oder deren Gemischen oder ohne Zusatz von Lösungsmitteln ablaufen. Die Ansätze sind in der Regel weitgehend wasserfrei (d.h. unter 10, bevorzugt unter 5 Vol% Wasserzusatz).

Der Anteil organischer Lösungsmittel beträgt beispielsweise 0,01-90 Gew.-%. Geeignete organische Lösungsmittel sind solche für diese Zwecke bekannten, beispielsweise tertiäre Monoole, wie C₃-C₆-Alkohole, bevorzugt tert-Butanol, tert-Amylalkohol, Pyridin, Poly-C₁-C₄-alkylenglykoldi-C₁-C₄-alkylether, bevorzugt Polyethylenglycoldi-C₁-C₄-alkylether, wie z.B. 1,2-Dimethoxyeethan, Diethylenglycoldimethylether, Polyethylenglycoldimethylether 500,
C₁-C₄-Alkylencarbonate, insbesondere Propylencarbonat, C₃-C₆-Alkylessigsäureester, insbesondere tert.-Butyl-essigsäureester, THF, Toluol, 1,3-Dioxolan, Aceton, *iso*-Butylmethylketon, Ethylmethylketon, 1,4-Dioxan, tert-Butylmethylether, Cyclohexan, Methylcyclohexan, Toluol, Hexan, Dimethoxymethan, 1,1-Dimethoxyethan, Acetonitril, sowie deren ein- oder mehrphasige Mischungen. Es kann vorteilhaft sein, Reaktionswasser durch ein möglichst nahe am Temperaturoptimum des verwendeten Enzyms siedende binäres oder ternäres Heteroazeotrop abzutrennen, beispielsweise Ethylmethylketon/Hexan/Wasser. Das azeotrop entfernte Wasser kann dann durch Phasenscheidung oder Membrandampftrennung entfernt werden.

Wahlweise können zu den organischen Lösungsmitteln wässrige Lösungsmittel zugesetzt werden, so dass - je nach organischem Lösungsmittel - ein- oder mehrphasige Reaktionslösungen entstehen. Beispiele für wässrige Lösungsmittel sind Wasser sowie wässrige, verdünnte (z.B. 10 bis 100mM) Puffer, beispielsweise mit einem pH-Wert im Bereich von etwa 6 bis 8, wie z.B. Kaliumphosphat- oder TRIS-HCl-Puffer.

Der Wasseranteil im Reaktionsansatz liegt in der Regel bei 0-10 Vol%. Bevorzugt werden die Reaktanden ohne Vorbehandlung (Trocknung, Wasserdotierung) eingesetzt.

Die Substrate liegen entweder gelöst, als Feststoffe suspendiert oder in Emulsion im Reaktionsmedium vor. Vorzugsweise liegt die anfängliche Konzentration der Reaktanden im Bereich von etwa 0,1 bis 20 Mol/l, insbesondere bei 0,15 bis 10 Mol/l oder 0,2 bis 5 mol/l liegt.

Die Reaktion kann kontinuierlich, beispielsweise in einem Rohrreaktor oder in einer Rührreaktorkaskade, oder diskontinuierlich erfolgen.

Die Umsetzung kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor oder einem Festbettreaktor.

Zur Durchmischung des Reaktionsansatzes können beliebige Verfahren eingesetzt werden. Spezielle Rührvorrichtungen sind nicht erforderlich. Das Reaktionsmedium kann ein- oder mehrphasig sein und die Reaktanden werden darin gelöst, suspendiert oder emulgiert, gegebenenfalls zusammen mit dem Molekularsieb vorgelegt und zum Start der Reaktion, sowie gegebenenfalls ein- oder mehrmals im Verlauf der Reaktion, mit dem Enzympräparat versetzt. Die Temperatur wird während der Reaktion auf den gewünschten Wert eingestellt und kann, falls gewünscht, während des Reaktionsverlauf erhöht oder verringert werden.

Wird die Reaktion im Festbettreaktor durchgeführt, so ist der Festbettreaktor bevorzugt mit immobilisierten Enzymen bestückt, wobei die Reaktionsmischung durch eine mit dem Enzym gefüllte Säule gepumpt wird. Es ist auch möglich, die Umsetzung im Wirbelbett durchzuführen, wobei das Enzym auf einem Träger immobilisiert eingesetzt wird. Die Reaktionsmischung kann kontinuierlich durch die Säule gepumpt werden, wobei mit der Fließgeschwindigkeit die Verweilzeit und damit der gewünschte Umsatz steuerbar ist. Es ist auch möglich, die Reaktionsmischung im Kreislauf durch eine Säule zu pumpen, wobei auch unter Vakuum das Reaktionswasser bzw. der freigesetzte Alkohol gleichzeitig abdestilliert werden kann.

Die Entfernung des Reaktionswassers bzw. von Alkoholen, die bei einer Umesterung aus den Alkylacrylaten freigesetzt werden, erfolgt kontinuierlich oder schrittweise in an sich bekannter Weise, z.B. durch Vakuum, azeotrope Entfernung, Absorption, Pervaporation und Diffusion über Membranen.

Hierzu eignen sich vorzugsweise Molekularsiebe (Porengröße z.B. im Bereich von etwa 3-10 Angström), eine Abtrennung durch Destillation oder mit Hilfe geeigneter semipermeabler Membranen.

Nach Beendigung der Reaktion kann man das aus c) erhältliche Reaktionsgemisch ohne weitere Aufreinigung weiterverwenden oder es erforderlichenfalls in einem weiteren Schritt d) aufreinigen.

d) In der Regel wird lediglich das eingesetzte Enzym sowie ggf. eingesetztes Molsieb vom Reaktionsgemisch abgetrennt und das Reaktionsprodukt vom gegebenenfalls verwendeten organischen Lösungsmittel abgetrennt.

Eine Abtrennung vom Enzym erfolgt in der Regel durch Filtration, Absorption, Zentrifugation oder Dekantieren. Das abgetrennte Enzym kann anschließend für weitere Reaktionen eingesetzt werden.

Die Abtrennung vom organischen Lösungsmittel erfolgt in der Regel durch Destillation, Rektifikation oder bei festen Reaktionsprodukten durch Filtration.

Zur weiteren Aufreinigung des Reaktionsproduktes kann auch eine Chromatographie durchgeführt werden.

Bevorzugt werden in Schritt d) jedoch lediglich das eingesetzte Enzym und das gegebenenfalls eingesetzte Lösungsmittel abgetrennt.

Die Reaktionsbedingungen bei der enzymatischen Um- oder Veresterung sind mild. Aufgrund der niedrigen Temperaturen und sonstigen milden Bedingungen wird die Bildung von Nebenprodukten in Schritt c) vermieden, die andernfalls zum Beispiel von chemischen Katalysatoren stammen können oder durch unerwünschte radikalische Polymerisation des eingesetzten (Meth)acrylats oder der eingesetzten (Meth)acrylsäure, die sonst nur durch Zugabe von Stabilisatoren verhindert werden kann. Bei der erfindungsgemäßen Reaktionsführung kann der (Meth)acrylverbindung (D) über den ohnehin enthaltenen Lagerstabilisator hinaus zusätzliche Stabilisator zugegeben werden, beispielsweise Hydrochinonmonomethylether, Phenothiazin, Phenole, wie z.B. 2-tert.-Butyl-4-methylphenol, 6-tert.-Butyl-2,4-dimethyl-phenol oder N-Oxyle, wie 4-Hydroxy-2,2,6,6-tetramethyl-piperidin-N-oxyl, 4-Oxo-2,2,6,6-tetramethyl-piperidin-N-oxyl, beispielsweise in Mengen von 50 bis 2000 ppm. Vorteilhaft wird die Ver- oder Umes terung in Gegenwart eines sauerstoffhaltigen Gases, bevorzugt Luft oder Luft-Stick stoff-Gemische, durchgeführt. Des weiteren kann der Enzymkatalysator unproblematisch vom Endprodukt entfernt werden. Des weiteren wird in der Regel keine wesentliche Spaltung der Urethangruppen durch enzymatische Verseifung festgestellt, man findet in der Regel weniger als 10 %, bevorzugt weniger als 5 % Nebenprodukte.

Durch das erfindungsgemäße Verfahren sind in einer besonders bevorzugten Ausführungsform urethangruppenhaltige (Meth)acrylsäureester (F) der Formel (II) zugänglich, 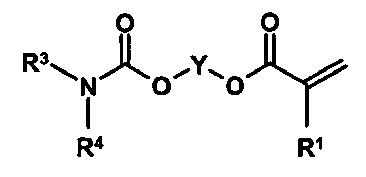 worin
- R³ und R⁴: die oben genannten Bedeutungen haben,
- Y: ausgewählt ist unter 1,2-Ethylen, 1,2-Propylen, 1,1-Dimethyl-1,2-ethylen, 1-Hydroxy-methyl-1,2-ethylen, 2-Hydroxy-1,3-propylen, 2-Hydroxy-1,3-propylen, 1,3-Propylen, 1,4-Butylen, 1,6-Hexylen, 2-Methyl-1,3-Propylen, 2-Ethyl-1,3-Propylen, 2,2-Dimethyl-1,3-Propylen und 2,2-Dimethyl-1,4-butylen, oder 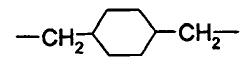 oder 1,2 -, 1,3- oder 1,4-Cyclohexylen,
- R¹: Wasserstoff oder Methyl, bevorzugt Wasserstoff bedeutet, mit der Maßgabe, daß mindestens einer der Reste R³ und R⁴ ungleich Wasserstoff ist.

Diese urethangruppenhaltigen (Meth)acrylsäureester zeigen in strahlungshärtbaren Beschichtungsmassen eine starke Erhöhung der Kratzfestigkeit und der Elastizität, bei einer gleichzeitig niedrigen Viskosität, was die Verbindungen der Formel (II) zu wertvollen Bestandteilen von strahlungshärtbaren Beschichtungsmassen macht.

Ein Vorteil des erfindungsgemäßen Verfahrens ist, daß im wesentlichen vollständige Umsätze mit einfachen (Meth)acrylverbindungen (D) erzielt werden können, da das Reaktionsgleichgewicht verschoben werden kann, ohne spezielle Edukte wie beispielsweise Vinylmethacrylat verwenden zu müssen.

Die aus den Stufen c) beziehungsweise d) erhältlichen urethangruppenhaltigen (Meth)-acrylsäureester können vorteilhaft als Comonomere in beispielsweise Poly(meth)-acrylaten oder als Reaktivverdünner in strahlungshärtbaren und/oder Dual-Cure-härtbaren Poly(meth)acrylaten eingesetzt werden. Derartige Poly(meth)acrylate sind als Bindemittel in strahlungs- oder Dual-Cure-härtbaren Beschichtungsmitteln geeignet. So erhältliche Beschichtungen weisen sehr hohe Kratzfestigkeiten, Härten, Chemikalienbeständigkeiten, Elastizität und Haftung, sowohl auf hydrophilen als auch auf hydrophoben Substraten auf.

Die erfindungsgemäß hergestellten urethangruppenhaltiger (Meth)acrylsäureester können aufgrund ihrer geringeren Färbung vorteilhaft auch in einer thermisch induzierten (radikalischen) (Co)polymerisation eingesetzt werden.

Als Monomere, mit denen die erfindungsgemäß hergestellten urethangruppenhaltiger (Meth)acrylsäureester beispielsweise copolymerisiert werden können seien genannt z.B. C₁-C₂₀-Alkyl(meth)acrylate, Vinylaromaten mit bis zu 20 C-Atomen, Vinylester von bis zu 20 C-Atomen enthaltenden Carbonsäuren, ethylenisch ungesättigte Nitrile, Vinylether von 1 bis 10 C-Atome enthaltenden Alkoholen und aliphatischen Kohlenwasserstoffen mit 2 bis 8 C-Atomen und 1 oder 2 Doppelbindungen.

Der Begriff (Meth)acrylsäure wird im Rahmen dieser Anmeldung für Acrylsäure und Methacrylsäure verwendet.

Als (Meth)acrylsäurealkylester bevorzugt sind solche mit einem C₁-C₁₀-Alkylrest, wie Methylmethacrylat, Methylacrylat, n-Butylacrylat, Ethylacrylat und verzweigte Alkylderivate wie 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, Vinylstearat, Vinylpropionat und Vinylacetat.

Als vinylaromatische Verbindungen kommen z.B. Vinyltoluol, α-Butylstyrol, 4-n-Butylstyrol, 4-n-Decylstyrol und vorzugsweise Styrol in Betracht.

Beispiele für Nitrile sind Acrylnitril und Methacrylnitril.

Geeignete Vinylether sind z.B. Vinylmethylether, Vinylisobutylether, Vinylhexylether und Vinyloctylether.

Als nicht aromatische Kohlenwasserstoffe mit 2 bis 8 C-Atomen und einer oder zwei olefinischen Doppelbindungen seien Butadien, Isopren, sowie Ethylen, Propylen und Isobutylen genannt.

Eine häufige, aber nicht die einzige Methode zur Herstellung solcher (Co)Polymerisate ist die radikalische oder ionische (Co)Polymerisation in einem Lösungs- oder Verdünnungsmittel.

Die radikalische (Co)Polymerisation solcher Monomere erfolgt beispielsweise in wäßriger Lösung in Gegenwart von Polymerisationsinitiatoren, die unter Polymerisationsbedingungen in Radikale zerfallen, beispielsweise Peroxodisulfate, H₂O₂Redoxysysteme oder Hydroxyperoxide, wie z.B. tert. Butylhydroperoxid oder Cumolhydroperoxid. Die (Co)Polymerisation kann in einem weiten Temperaturbereich, gegebenenfalls unter vermindertem oder auch unter erhöhtem Druck in der Regel bei Temperaturen bis zu 100 °C vorgenommen werden. Der pH-Wert des Reaktionsgemisches wird gewöhnlich in dem Bereich von 4 bis 10 eingestellt.

Die (Co)Polymerisation kann aber auch in anderer, dem Fachmann an sich bekannter Weise kontinuierlich oder diskontinuierlich durchgeführt werden, z.B. als Lösungs-, Fällungs-, Wasser-in-Öl-Emulsions-, inverse Emulsions, Suspensions oder umgekehrte Suspensionspolymerisation.

Dabei wird das Monomer/die Monomere unter Verwendung radikalischer Polymerisationsinitiatoren, z.B. in Radikale zerfallende Azoverbindungen, wie 2,2'-Azo-bis(iso-butyronitril), 2,2'-Azobis-(2-amidinopropan)-hydrochlorid oder 4,4'-Azo-bis-(4'-cyanpentansäure) oder Dialkylperoxide, wie Di-tert.-Amylperoxid, Aryl-alkylperoxide, wie tert.-Butyl-cumylperoxid, Alkyl-acylperoxide, wie tert.-Butyl-peroxy-2-ethylhexanoat, Peroxidicarbonate, wie Di-(4-tert.-Butylcyclohexyl)peroxydicarbonat oder Hydroperoxide (co)polymerisiert.

Die genannten Verbindungen werden meist in Form wäßriger Lösungen oder wäßriger Emulsionen eingesetzt, wobei die untere Konzentration durch die in der (Co)Polymerisation vertretbare Wassermenge und die obere Konzentration durch die Löslichkeit der betreffenden Verbindung in Wasser bestimmt ist.

Als Lösungs- oder Verdünnungsmittel können dienen z.B. Wasser, Alkohole, wie Methanol, Ethanol, n- oder iso-Propanol, n- oder iso-Butanol, oder Ketone, wie Aceton, Ethylmethylketon, Diethylketon oder *iso*-Butylmethylketon. Besonders bevorzugt sind unpolare Lösungsmittel wie beispielsweise Xylol und dessen Isomerengemische, Shellsol® A und Solventnaphtha.

In einer bevorzugten Ausführungsform werden die Monomere vorgemischt und Initiator mit gegebenenfalls weiteren Zusätzen gelöst in Lösungsmittel zugegeben. Eine besonders bevorzugte Ausführungsform ist beschrieben in WO 01/23484 und dort besonders auf Seite 10, Z. 3 bis Z. 24

Gegebenenfalls kann die (Co)Polymerisation in Gegenwart von Polymerisationsreglern, wie beispielsweise Hydroxylammoniumsalze, chlorierte Kohlenwasserstoffe und Thioverbindungen, wie z.B. tert.-Butylmercaptan, Thioglycolsäureethylacrylester, Mercaptoethynol, Mercaptopropyltrimethoxysilan, Dodecylmercaptan, tert.-Dodecylmercaptan oder Alkalimetallhypophosphite, durchgeführt werden. Bei der (Co)Polymerisation können diese Regler, z. B. in Mengen von 0 bis 0,8 Gew.-Teile, bezogen auf 100 Gew.-Teile der zu (co)polymerisierenden Monomeren, eingesetzt werden, durch die die Molmasse des entstehenden (Co)Polymers verringert wird.

Bei der Emulsionspolymerisation können Dispergiermittel, ionische und/oder nichtionische Emulgatoren und/oder Schutzkolloide bzw. Stabilisatoren als grenzflächenaktive Verbindungen verwendet werden. Als solche kommen sowohl die zur Durchführung von Emulsionspolymerisationen üblicherweise eingesetzten Schutzkolloide als auch Emulgatoren in Betracht.

Geeignete Schutzkolloide sind beispielsweise Polyvinylalkohole, Cellulosederivate oder Vinylpyrrolidon enthaltende Copolymerisate. Eine ausführliche Beschreibung weiterer geeigneter Schutzkolloide findet sich in Houben-Weyl, Methoden der organischen Chemie, Band XIV/1, makromolekulare Stoffe, Georg-Thieme-Verlag, Stuttgart, 1969, S. 411 bis 420. Selbstverständlich können auch Gemische aus Emulgatoren und/oder Schutzkolloiden verwendet werden. Vorzugsweise werden als Dispergiermittel ausschließlich Emulgatoren eingesetzt, deren relative Molekulargewichte im Unterschied zu den Schutzkolloiden üblicherweise unter 1000 liegen. Sie können sowohl anionischer, kationischer oder nichtionischer Natur sein. Selbstverständlich müssen im Falle der Verwendung von Gemischen granzflächenaktiver Substanzen die Einzelkomponenten miteinander verträglich sein, was im Zweifelsfall an Hand weniger Vorversuche überprüft werden kann. Im allgemeinen sind anionische Emulgatoren untereinander und mit nichtionischen Emulgatoren verträglich.

Desgleichen gilt auch für kationische Emulgatoren, während anionische und kationische Emulgatoren meistens miteinander unverträglich sind. Gebräuchliche Emulgatoren sind z.B. ethoxylierte Mono-, Di- und Tri-Alkylphenole (EO-Grad: 3 bis 100, : C₄ bis (C₁₂), ethoxylierte Fettalkohole (EO-Grad: 3 bis 100, Alkylrest: C₈ bis C₁₈), sowie Alkali- und Ammoniumsalze von Alkylsulfaten (Alkylrest: C₈ bis C₁₆) von Schwefelsäurehalbestern ethoxylierter Alkylphenole (EO-Grad: 3 bis 100, Alkylrest: C₄ bis C₁₂), von Alkylsulfonsäuren (Alkylrest: C₁₂ bis C₁₈) und von Alkylacrylsulfonsäuren (Alkylrest: C₉ bis C₁₈). Weitere geeignete Emulgatoren wie Sulfobemsteinsäureester finden sich in Houben-Weyl, Methoden der organischen Chemie, Band XIV/1, Makromolekulare Stoffe, Georg-Thieme Verlag, Stuttgart, 1961, Seiten 192 bis 208.

In der Regel beträgt die Menge an eingesetzten Dispergiermittel 0,5 bis 6, vorzugsweise 1 bis 3 Gew.-% bezogen auf die radikalisch zu polymerisierenden Monomeren.

Beispiele für (meth)acrylathaltige Dispersionen sind n-Butylacrylat/Acrylnitril - Dispersionen, die als Klebstoffe Anwendung finden, n-Butylacrylat/Butadien/Styrol-

Die Polymerdispersionen, in denen erfindungsgemäß hergestellte urethangruppenhaltige (Meth)acrylsäureester verwendet werden, können zusätzlich chemisch und/oder physikalisch desodoriert werden.

Die mit den erfindungsgemäß hergestellten urethangruppenhaltige (Meth)acrylsäureester erhältlichen Copolymerisate weisen in der Regel eine geringere Farbzahl auf, was im Lackbereich vorteilhaft ist. Die beschriebenen Copolymerisate lassen sich dann in an sich bekannter Weise beispielsweise mit Aminoplasten, wie z.B. Melamin, zu vernetzten Lackharzen umsetzen, wie es beispielsweise beschrieben ist in der EP 738740 oder EP 675141.

Ein weiterer Gegenstand der vorliegenden Anmeldung ist daher die Verwendung der nach dem erfindungsgemäßen Verfahren hergestellten urethangruppenhaltigen (Meth)-acrylsäureester als Reaktiwerdünner oder Bindemittel in Strahlen oder Dual-Cure-härtbaren Beschichtungsmassen, bevorzugt in Deckbeschichtungen, besonders bevorzugt in transparenten Klarlacken. Selbstverständlich können die erfindungsgemäß hergestellten urethangruppenhaltigen (Meth)acrylsäureester auch als Monomere in Polymerisationen, gegebenenfalls zusammen mit anderen polymerisierbaren Monomeren, wie z.B. (Meth)acrylsäure, (Meth)acrylsäureester, Styrol, Butadien, Acrylnitril, Vinylacetat, N-Vinylpyrrolidon, 4-Hydroxybutylvinylether oder N-Vinylformamid, verwendet werden.

Unter "Dual-Cure" ist zu verstehen, dass die Beschichtungsmassen thermisch und mit aktinischer Strahlung härtbar sind. Im Rahmen der vorliegenden Erfindung ist unter aktinischer Strahlung elektromagnetische Strahlung wie sichtbares Licht, UV-Strahlung oder Röntgenstrahlung, insbesondere UV-Strahlung, und Korpuscularstrahlung wie Elektronenstrahlung zu verstehen.

Strahlenhärtbare Bindemittel sind solche, die mittels aktinischer Strahlung wie vorstehend definiert, insbesondere mittels UV-Strahlung härtbar sind.

Ein weiterer Gegenstand der vorliegenden Anmeldung sind Lackformulierungen, enthaltend die nach dem erfindungsgemäßen Verfahren erhältlichen urethangruppenhaltigen (Meth)acrylsäureester. Dabei können die urethangruppenhaltigen (Meth)acrylsäureester sowohl in Basislacken als auch in Decklacken eingesetzt werden. Aufgrund ihrer besonderen Eigenschaften, wie der Erhöhung der Kratzfestigkeit und Elastizität, sowie der Erniedrigung der Viskosität, insbesondere bei verzweigten Polyacrylaten, einer strahlengehärteten Klarlackbeschichtung, ist ihr Einsatz in Deckbeschichtungen bevorzugt.

Neben den nach dem erfindungsgemäßen Verfahren erhältlichen urethangruppenhaltigen (Meth)acrylsäureester (F) kann eine erfindungsgemäße strahlungshärtbare Masse noch folgende Komponenten enthalten:
(G) mindestens eine polymerisierbare Verbindung mit mehreren copolymerisierbaren, ethylenisch ungesättigten Gruppen,
(H) gegebenenfalls Reaktivverdünner,
(1) gegebenenfalls Photoinitiator sowie
(J) gegebenenfalls weitere lacktypische Additive.

Als Verbindungen (G) kommen strahlungshärtbare, radikalisch polymerisierbare Verbindungen mit mehreren, d.h. mindestens zwei, copolymerisierbaren, ethylenisch ungesättigten Gruppen in Betracht.

Bevorzugt handelt es sich bei Verbindungen (G) um Vinylether- oder (Meth)acrylatverbindungen, besonders bevorzugt sind jeweils die Acrylatverbindungen, d.h. die Derivate der Acrylsäure.

Bevorzugte Vinylether- und (Meth)acrylat-Verbindungen (G) enthalten 2 bis 20, bevorzugt 2 bis 10 und ganz besonders bevorzugt 2 bis 6 copolymerisierbare, ethylenisch ungesättigte Doppelbindungen.

Besonders bevorzugt sind solche Verbindungen (G) mit einem Gehalt an ethylenisch ungesättigte Doppelbindungen von 0,1 -0,7 mol /100 g, ganz besonders bevorzugt 0,2 - 0,6 mol / 100 g.

Das zahlenmittlere Molekulargewicht Mₙ der Verbindungen (G) liegt, wenn nicht anders angegeben, bevorzugt unter 15000, besonders bevorzugt bei 300 -12000, ganz besonders bevorzugt bei 400 bis 5000 und insbesondere bei 500 - 3000 g/mol (bestimmt durch Gelpermeationschromatographie mit Polystyrol als Standard und Tetrahydrofuran als Elutionsmittel).

Als (Meth)acrylatverbindungen genannt seien (Meth)acrylsäureester und insbesondere Acrylsäureester sowie Vinylether von mehrfunktionellen Alkoholen, insbesondere solchen, die neben den Hydroxylgruppen keine weiteren funktionellen Gruppen oder allenfalls Ethergruppen enthalten. Beispiele solcher Alkohole sind z.B. bifunktionelle Alkohole, wie Ethylenglykol, Propylenglykol und deren höher kondensierte Vertreter, z.B. wie Diethylenglykol, Triethylenglykol, Dipropylenglykol, Tripropylenglykol etc., 1,2-, 1,3-oder 1,4-Butandiol, 1,5-Pentandiol, 1,6-Hexandiol,
3-Methyl-1,5-pentandiol, Neopentylglykol, alkoxylierte phenolische Verbindungen, wie ethoxylierte bzw. propoxylierte Bisphenole, 1,2-, 1,3- oder 1,4-Cyclohexandimethanol, trifunktionelle und höherfunktionelle Alkohole, wie Glycerin, Trimethylolpropan, Butantriol, Trimethylolethan, Pentaerythrit, Ditrimethylolpropan, Dipentaerythrit, Sorbit, Mannit und die entsprechenden alkoxylierten, insbesondere ethoxylierten und/oder propoxylierten Alkohole.

Die Alkoxylierungsprodukte sind in bekannter Weise durch Umsetzung der vorstehenden Alkohole mit Alkylenoxiden, insbesondere Ethylen- oder Propylenoxid, erhältlich. Vorzugsweise beträgt der Alkoxylierungsgrad je Hydroxylgruppe 0 bis 10, d.h. 1 mol Hydroxylgruppe kann mit bis zu 10 mol Alkylenoxiden alkoxyliert sein.

Als (Meth)acrylatverbindungen seien weiterhin Polyester(meth)acrylate genannt, wobei es sich um die (Meth)acrylsäureester oder Vinylether von Polyesterolen handelt, sowie Urethan-, Epoxid- oder Melamin(meth)acrylate.

Urethan(meth)acrylate sind z.B. erhältlich durch Umsetzung von Polyisocyanaten mit Hydroxyalkyl(meth)acrylaten und gegebenenfalls Kettenverlängerungsmitteln wie Diolen, Polyolen, Diaminen, Polyaminen oder Dithiolen oder Polythiolen.

Die Urethan(meth)acrylate haben vorzugsweise ein zahlenmittleres Molgewicht Mₙ von 500 bis 20 000, insbesondere von 750 bis 10 000 besonders bevorzugt 750 bis 3000 g/mol (bestimmt durch Gelpermeationschromatographie mit Polystyrol als Standard).

Die Urethan(meth)acrylate haben vorzugsweise einen Gehalt von 1 bis 5, besonders bevorzugt von 2 bis 4 Mol (Meth)acrylgruppen pro 1000 g Urethan(meth)acrylat.

Epoxid(meth)acrylate sind erhältlich durch Umsetzung von Epoxiden mit (Meth)acrylsäure. Als Epoxide in Betracht kommen z.B epoxidierte Olefine oder Glycidylether, z.B. Bisphenol-A-diglycidylether oder aliphatische Glycidylether, wie Butandioldiglycidether.

Melamin(meth)acrylate sind erhältlich durch Umsetzung von Melamin mit (Meth)acrylsäure oder deren Ester.

Die Epoxid(meth)acrylate und Melamin(meth)acrylate haben vorzugsweise ein zahlenmittleres Molgewicht Mₙ von 500 bis 20000, besonders bevorzugt von 750 bis 10000 g/mol und ganz besonders bevorzugt von 750 bis 3000 g/mol; der Gehalt an (Meth)acrylgruppen beträgt vorzugsweise 1 bis 5, besonders bevorzugt 2 bis 4 pro 1000 g Epoxid(meth)acrylat oder Melamin(meth)acrylat (bestimmt durch Gelpermeationschromatographie mit Polystyrol als Standard und Tetrahydrofuran als Elutionsmittel).

Weiterhin geeignet sind Carbonat(meth)acrylate, die im Mittel vorzugsweise 1 bis 5, insbesondere 2 bis 4, besonders bevorzugt 2 bis 3 (Meth)acrylgruppen und ganz besonders bevorzugt 2(Meth)acrylgruppen enthalten.

Das zahlungsmittlere Molekulargewicht Mₙ der Carbonat(meth)acrylate ist vorzugsweise kleiner 3000 g/mol, besonders bevorzugt kleiner 1500 g/mol, besonders bevorzugt kleiner 800 g/mol (bestimmt durch Gelpermeationschromatgraphie mit Polystyrol als Standard, Lösemittel Tetrahydrofuran).

Die Carbonat(meth)acrylate sind in einfacher Weise erhältlich durch Umesterung von Kohlensäureestern mit mehrwertigen, vorzugsweise zweiwertigen Alkoholen (Diolen, z.B. Hexandiol) und anschließende Veresterung der freien OH-Gruppen mit (Meth)-acrylsäure oder auch Um-esterung mit (Meth)acrylsäureestern, wie es z.B. in EP-A 92 269 beschrieben ist. Erhältlich sind sie auch durch Umsetzung von Phosgen, Harnstoffderivaten mit mehrwertigen, z.B. zweiwertigen Alkoholen.

Als Reaktiwerdünner (Verbindungen (H)) kommen strahlungshärtbare, radikalisch oder kationisch polymerisierbare Verbindungen mit nur einer ethylenisch ungesättigten, copolymerisierbaren Gruppe in Betracht.

Genannt seien z.B. C₁-C₂₀-Alkyl(meth)acrylate, Vinylaromaten mit bis zu 20 C-Atomen, Vinylester von bis zu 20 C-Atomen enthaltenden Carbonsäuren, ethylenisch ungesättigte Nitrile, Vinylether von 1 bis 10 C-Atome enthaltenden Alkoholen, α,β-ungesättigte Carbonsäuren und deren Anhydride und aliphatischen Kohlenwasserstoffen mit 2 bis 8 C-Atomen und 1 oder 2 Doppelbindungen.

Als (Meth)acrylsäurealkylester bevorzugt sind solche mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, Vinylstearat, Vinylpropionat und Vinylacetat.

α,β-Ungesättigte Carbonsäuren und deren Anhydride können beispielsweise sein Acrylsäure, Methacrylsäure, Fumarsäure, Crotonsäure, Itaconsäure, Maleinsäure oder Maleinsäureanhydrid, bevorzugt Acrylsäure.

Als vinylaromatische Verbindungen kommen z.B. Vinyltoluol, α-Butylstyrol, 4-n-Butylstyrol, 4-n-Decylstyrol und vorzugsweise Styrol in Betracht.

Beispiele für Nitrile sind Acrylnitril und Methacrylnitril.

Geeignete Vinylether sind z.B. Vinylmethylether, Vinylisobutylether, Vinylhexylether und Vinyloctylether.

Als nicht aromatische Kohlenwasserstoffe mit 2 bis 8 C-Atomen und einer oder zwei olefinischen Doppelbindungen seien Butadien, Isopren, sowie Ethylen, Propylen und Isobutylen genannt.

Weiterhin sind N-Vinylformamid, N-Vinylpyrrolidon sowie N-Vinylcaprolactam einsetzbar.

Als Photoinitiatoren (I) können dem Fachmann bekannte Photoinitiatoren verwendet werden, z.B. solche in "Advances in Polymer Science", Volume 14, Springer Berlin 1974 oder in K. K. Dietliker, Chemistry and Technology of UV- and EB-Formulation for Coatings, Inks and Paints, Volume 3; Photoinitiators for Free Radical and Cationic Polymerization, P. K. T. Oldring (Eds), SITA Technology Ltd, London, genannten.

In Betracht kommen z.B. Mono- oder Bisacylphosphinoxide Irgacure 819 (Bis(2,4,6-Trimethylbenzoyl)phenylphosphinoxid), wie sie z.B. in EP-A 7 508, EP-A 57 474, DE-A 19618 720, EP-A 495 751 oder EP-A 615 980 beschrieben sind, beispielsweise 2,4,6-Trimethylbenzoyldiphenylphosphinoxid (Lucirin^{®} TPO), Ethyl-2,4,6-trimethylbenzoylphenylphosphinat, Benzophenone, Hydroxyacetophenone, Phenylglyoxylsäure und ihre Derivate oder Gemische dieser Photoinitiatoren. Als Beispiele seien genannt Benzophenon, Acetophenon, Acetonaphthochinon, Methylethylketon, Valerophenon, Hexanophenon, α-Phenylbutyrophenon, p-Morpholinopropiophenon, Dibenzosuberon, 4-Morpholinobenzophenon, 4-Morpholinodeoxybenzoin, p-Diacetylbenzol, 4-Aminobenzophenon, 4'-Methoxyacetophenon β-Methylanthrachinon, *tert*-Butylanthrachinon, Anthrachinoncarbonysäureester, Benzaldehyd, α-Tetralon, 9-Acetylphenanthren, 2-Acetylphenanthren, 10-Thioxan-thenon, 3-Acetylphenanthren, 3-Acetylindol, 9-Fluorenon, 1-Indanon, 1,3,4-Triacetylbenzol, Thioxanthen-9-on, Xanthen-9-on, 2,4-Dimethylthioxanthon, 2,4-Diethylthioxanthon, 2,4-Di-iso-propylthioxanthon, 2,4-Dichlorthioxanthon, Benzoin, Benzoin-iso-butylether, Chloroxanthenon, Benzointetrahydropyranylether, Benzoin-methylether, Benzoin-ethylether, Benzoin-butylether, Benzoin-iso-propylether, 7-H-Benzoin-methylether, Benz[de]anthracen-7-on, 1-Naphthaldehyd, 4,4'-Bis(dimethylamino)benzophenon, 4-Phenylbenzophenon, 4-Chlorbenzophenon, Michlers Keton, 1-Acetonaphthon, 2-Acetonaphthon, 1-Benzoylcyclohexan-1-ol, 2-Hydroxy-2,2-dimethylacetophenon, 2,2-Dimethoxy-2-phenylacetophenon, 2,2-Diethoxy-2-phenylacetophenon, 1,1-Dichloracetophenon, 1-Hydroxyacetophenon, Acetophenondimethylketal, o-Methoxybenzo-phenon, Triphenylphosphin, Tri-o-Tolylphosphin, Benz[a]anthracen-7,12-dion, 2,2-Diethoxy-acetophenon, Benzilketale, wie Benzildimethylketal, 2-Methyl-1-[4-(methylthio)phenyl]-2-mor-pholinopropan-1-on, Anthrachinone wie 2-Methylanthrachinon, 2-Ethylanthrachinon, 2-t*ert*-Bu-tylanthrachinon, 1-Chloranthrachinon, 2-Amylanthrachinon und 2,3-Butandion.

Geeignet sind auch nicht- oder wenig vergilbende Photoinitiatoren vom Phenylglyoxalsäureestertyp, wie in DE-A 198 26 712, DE-A 19913 353 oder WO 98/33761 beschrieben.

Unter den genannten Photoinitiatoren sind Phosphinoxide, α-Hydroxyketone und Benzophenone bevorzugt.

Insbesondere können auch Gemische verschiedener Photoinitiatoren verwendet werden.

Die Photoinitiatoren können allein oder in Kombination mit einem Photopolymerisationspromotor, z.B. vom Benzoesäure-, Amin- oder ähnlichem Typ verwendet werden.

Als weitere lacktypische Additive (J) können beispielsweise Antioxidantien, Oxidationsinhibitoren, Stabilisatoren, Aktivatoren (Beschleuniger), Füllmittel, Pigmente, Farbstoffe, Entgasungsmittel, Glanzmittel, antistatische Agentien, Flammschutzmittel, Verdicker, thixotrope Agentien, Verlaufshilfsmittel, Bindemittel, Antischaummittel, Duftstoffe, oberflächenaktive Agentien, Viskositätsmodifikatoren, Weichmacher, Plastifizierer, klebrigmachende Harze (Tackifier), Chelatbildner oder Verträglichkeitsmittel (compatibilizer) verwendet werden.

Als Beschleuniger für die thermische Nachhärtung kann z.B. Zinnoctoat, Zinkoctoat, Dibutylzinnlaureat oder Diaza[2.2.2]bicyclooctan verwendet werden.

Weiterhin können ein oder mehrere photochemisch und/oder thermisch aktivierbare Initiatoren zugesetzt werden, z.B. Kaliumperoxodisulfat, Dibenzoylperoxid, Cyclohexanonperoxid, Di-tert.-Butylperoxid, Azobis-*iso*-butyronitril, Cyclohexylsulfonylacetylperoxid, Di-iso-propylpercarbonat, *tert*-Butylperoktoat oder Benzpinakol, sowie beispielsweise solche thermisch aktivierbare Initiatoren, die eine Halbwertszeit bei 80°C von mehr als 100 Stunden aufweisen, wie Di-t-Butyl-peroxid, Cumolhydroperoxid, Dicumylperoxid, t-Butylperbenzoat, silylierte Pinakole, die z. B. unter dem Handelsnamen ADDID 600 der Firma Wacker kommerziell erhältlich sind oder Hydroxylgruppenhaltige Amin-N-Oxide, wie 2,2,6,6-Tetramethylpiperidin-N-oxyl, 4-Hydroxy-2,2,6,6-Tetramethylpiperidin-N-oxyl etc.

Weitere Beispiele geeigneter Initiatoren sind in "Polymer Handbook", 2. Aufl., Wiley & Sons, New York beschrieben.

Als Verdicker kommen neben radikalisch (co)polymerisierten (Co)Polymerisaten, übliche organische und anorganische Verdicker wie Hydroxymethylcellulose oder Bentonite in Betracht.

Als Chelatbildner können z.B. Ethylendiaminessigsäure und deren Salze sowie β-Diketone verwendet werden.

Geeignete Füllstoffe umfassen Silikate, z. B. durch Hydrolyse von Siliciumtetrachlorid erhältliche Silikate wie Aerosil^{®} der Fa. Degussa, Kieselerde, Talkum, Aluminiumsilikate, Magnesiumsilikate, Calciumcarbonate etc.

Geeignete Stabilisatoren umfassen typische UV-Absorber wie Oxanilide, Triazine und Benzotriazol (letztere erhältlich als Tinuvin^{®} -Marken der Ciba-Spezialitätenchemie) und Benzophenone. Diese können allein oder zusammen mit geeigneten Radikalfängern, beispielsweise sterisch gehinderten Aminen wie 2,2,6,6-Tetramethylpiperidin, 2,6-Di-tert.-butylpiperidin oder deren Derivaten, z. B. Bis-(2,2,6,6-tetra-methyl-4-piperidyl)sebacinat, eingesetzt werden. Stabilisatoren werden üblicherweise in Mengen von 0,1 bis 5,0 Gew.-%, bezogen auf die in der Zubereitung enthaltenen festen Komponenten, eingesetzt.

Weiterhin geeignete Stabilisatoren sind beispielsweise N-Oxyle, wie z.B. 4-Hydroxy-2,2,6,6-te-tramethyl-piperidin-N-oxyl, 4-Oxo-2,2,6,6-tetramethyl-piperidin-N-oxyl, 4-Acetoxy-2,2,6,6-te-tramethyl-piperidin-N-oxyl, 2,2,6,6-tetramethyl-piperidin-N-oxyl, 4,4',4"-Tris(2,2,6,6-tetramethyl-piperidin-N-oxyl)-phosphit oder 3-Oxo-2,2,5,5-tetramethyl-pyrrolidin-N-oxyl, Phenole und Naphthole, wie z.B. p-Aminophenol, p-Nitrosophenol, 2-*tert*.-Butylphenol, 4-*tert*.-Butylphenol, 2,4-di-*tert*.-Butylphenol, 2-Methyl-4-*tert*.-Butylphenol, 4-Methyl-2,6-*tert*.-Butylphenol (2,6-*tert*.-Butyl-p-Kresol) oder 4*-tert.-*Butyl-2,6-dimethylphenol, Chinone, wie z.B. Hydrochinon oder Hydrochinonmonomethylether, aromatische Amine, wie z.B. N,N-Diphenylamin, N-Nitroso-diphenylamin, Phenylendiamine, wie z.B. N,N'-Dialkyl-para-phenylendiamin, wobei die Alkylreste gleich oder verschieden sein können und jeweils unabhängig voneinander aus 1 bis 4 Kohlenstoffatome bestehen und geradkettig oder verzweigt sein können, Hydroxylamine, wie z.B.
N,N-Diethylhydroxylamin, Harnstoffderivate, wie z.B. Harnstoff oder Thioharnstoff, phosphorhaltige Verbindungen, wie z.B. Triphenylphosphin, Triphenylphosphit oder Triethylphosphit oder schwefelhaltige Verbindungen, wie z.B. Diphenylsulfid oder Phenothiazin.

Typische Zusammensetzungen für strahlungshärtbare Massen sind beispielsweise
(F) 20 -100 Gew.-%, bevorzugt 40 - 90, besonders bevorzugt 50 - 90 und insbesondere
   60 - 80 Gew.-%,
(G) 0 - 60 Gew.-%, bevorzugt 5 - 50, besonders bevorzugt 10 - 40 und insbesondere
   10 - 30 Gew.-%,
(H) 0 - 50 Gew.%, bevorzugt 5 - 40, besonders bevorzugt 6 - 30 und insbesondere
   10 - 30 Gew.-%,
(I) 0 - 20 Gew.-%, bevorzugt 0,5 -15, besonders bevorzugt 1 -10 und insbesondere
   2 - 5 Gew.-% sowie
(J) 0 - 50 Gew.-%, bevorzugt 2 - 40, besonders bevorzugt 3 - 30 und insbesondere
   5 - 20 Gew.-%,
mit der Maßgabe, daß (F), (G), (H), (I) und (J) zusammen 100 Gew.-% ergeben.

Die Beschichtung der Substrate erfolgt nach üblichen, dem Fachmann bekannten Verfahren, wobei man wenigstens eine Beschichtungsmasse auf das zu beschichtende Substrat in der gewünschten Stärke aufbringt und die gegebenenfalls enthaltenen flüchtigen Bestandteile der Bechichtungsmasse, gegebenenfalls unter Erhitzen, entfernt. Dieser Vorgang kann gewünschtenfalls ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Coextrudieren erfolgen. Die Beschichtungsstärke liegt in der Regel in einem Bereich von etwa 3 bis 1000 g/m² und vorzugsweise 10 bis 200 g/m².

Weiterhin wird ein Verfahren zum Beschichten von Substraten offenbart, bei dem man die Beschichtungsmasse auf das Substrat aufbringt und gegebenenfalls trocknet, mit Elektronenstrahlen oder UV Belichtung unter sauerstoffhaltiger Atmosphäre oder bevorzugt unter Inertgas härtet, gegebenenfalls bei Temperaturen bis zur Höhe der Trocknungstemperatur und anschließend bei Temperaturen bis zu 160°C, bevorzugt zwischen 60 und 160 °C, thermisch behandelt.

Das Verfahren zum Beschichten von Substraten kann auch so durchgeführt werden, daß nach dem Aufbringen der Beschichtungsmasse zunächst bei Temperaturen bis zu 160°C, bevorzugt zwischen 60 und 160 °C, thermisch behandelt und anschließend mit Elektronenstrahlen oder UV Belichtung unter Sauerstoff oder bevorzugt unter Inertgas gehärtet wird.

Die Härtung der auf dem Substrat gebildeten Filme kann gewünschtenfalls ausschließlich thermisch erfolgen. Im allgemeinen härtet man die Beschichtungen jedoch sowohl durch Bestrahlung mit energiereicher Strahlung als auch thermisch.

Die Härtung kann auch zusätzlich oder anstelle der thermischen Härtung durch NIR-Strahlung erfolgen, wobei als NIR-Strahlung hier elektromagnetische Strahlung im Wellenlängenbereich von 760 nm bis 2,5 µm, bevorzugt von 900 bis 1500 nm bezeichnet ist.

Gegebenenfalls kann, wenn mehrere Schichten des Beschichtungsmittels übereinander aufgetragen werden, nach jedem Beschichtungsvorgang eine thermische, NIR und/oder Strahlungshärtung erfolgen.

Als Strahlungsquellen für die Strahlungshärtung geeignet sind z.B. Quecksilber-Niederdruckstrahler, -Mitteldruckstrahler mit Hochdruckstrahler sowie Leuchtstoffröhren, Impulsstrahler, Metallhalogenidstrahler, Elektronenblitzeinrichtungen, wodurch eine Strahlungshärtung ohne Photoinitiator möglich ist, oder Excimerstrahler. Die Strahlungshärtung erfolgt durch Einwirkung energiereicher Strahlung, also UV-Strahlung oder Tageslicht, vorzugsweise Licht im Wellenlängenbereich von λ=200 bis 700 nm strahlt, besonders bevorzugt von λ=200 bis 500 nm und ganz besonders bevorzugt λ=250 bis 400 nm, oder durch Bestrahlung mit energiereichen Elektronen (Elektronenstrahlung; 150 bis 300 keV). Als Strahlungsquellen dienen beispielsweise Hochdruckquecksilberdampflampen, Laser, gepulste Lampen (Blitzlicht), Halogenlampen oder Excimerstrahler. Die üblicherweise zur Vernetzung ausreichende Strahlungsdosis bei UV-Härtung liegt im Bereich von 80 bis 3000 mJ/cm².

Selbstverständlich sind auch mehrere Strahlungsquellen für die Härtung einsetzbar, z.B. zwei bis vier.

Diese können auch in jeweils unterschiedlichen Wellenlängebereichen strahlen.

Die Bestrahlung kann gegebenenfalls auch unter Ausschluß von Sauerstoff, z. B. unter Inertgas-Atmosphäre, durchgeführt werden. Als Inertgase eignen sich vorzugsweise Stickstoff, Edelgase, Kohlendioxid, oder Verbrennungsgase. Desweiteren kann die Bestrahlung erfolgen, indem die Beschichtungsmasse mit transparenten Medien abgedeckt wird. Transparente Medien sind z. B. Kunststofffolien, Glas oder Flüssigkeiten, z. B. Wasser. Besonders bevorzugt ist eine Bestrahlung in der Weise, wie sie in der DE-A1 199 57 900 beschrieben ist.

Ein weiterer Gegenstand der Erfindung ist ein Verfahren zur Beschichtung von Substraten, wobei man
i) ein Substrat mit einer Beschichtungsmasse, wie zuvor beschrieben, beschichtet,
ii) flüchtige Bestandteile der Beschichtungsmasse zur Filmbildung unter Bedingungen entfernt, bei denen der Photoinitiator (I) im wesentlichen noch keine freien Radikale ausbildet,
iii) gegebenenfalls den in Schritt ii) gebildeten Film mit energiereicher Strahlung bestrahlt, wobei der Film vorgehärtet wird, und anschließend gegebenenfalls den mit dem vorgehärteten Film beschichteten Gegenstand mechanisch bearbeitet oder die Oberfläche des vorgehärteten Films mit einem anderen Substrat in Kontakt bringt,
iv) dem Film thermisch oder mit NIR-Strahlung endhärtet

Dabei können die Schritte iv) und iii) auch in umgekehrter Reihenfolge durchgeführt, d. h. der Film kann zuerst thermisch oder per NIR-Strahlung und dann mit energiereicher Strahlung gehärtet werden.

Weiterhin sind auch Substrat, beschichtet mit einer erfindungsgemäßen Mehrschichtlackierung, Gegenstand der vorliegenden Erfindung.

Die Dicke einer solche wie beschrieben zu härtenden Schicht kann von 0,1 µm bis mehrere mm betragen, bevorzugt von 1 bis 2000 µm, besonders bevorzugt 5 bis 1000 µm, ganz besonders bevorzugt von 10 bis 500 µm und insbesondere von 10 bis 250 µm.

Besonders bevorzugt eignen sich die erfindungsgemäßen Beschichtungsmassen als oder in Außenbeschichtungen, also solche Anwendungen, die dem Tageslicht ausgesetzt sind, bevorzugt von Gebäuden oder Gebäudeteilen, Innenbeschichtungen, Straßenmarkierungen, Beschichtungen auf Fahrzeugen und Flugzeugen. Insbesondere werden die Beschichtungen als Holz-, Papier- oder Kunststoffbeschichtungen, beispielsweise für Parkett oder Möbel eingesetzt.

Die folgenden Beispiele sollen die Eigenschaften der Erfindung erläutern, ohne sie aber einzuschränken.

### Beispiele

Als "Teile" seien in dieser Schrift, wenn nicht anders angegeben, "Gewichtsteile" verstanden.

### Beispiel 1

Eine Mischung aus 2,5 mol (220 g) Ethylencarbonat und 2,5 mol (180 g) n-Butylamin wurde 2 h bei 100°C gerührt. Das Reaktionsprodukt wurde abfiltriert und 398 g Rohprodukt (klare, farblose Flüssigkeit) erhalten.

Der Umsatz, bestimmt mittels GC, betrug 99 %.

### Beispiel 2

Eine Mischung aus 2,5 mol (220g) Ethylencarbonat, 2,5 mol (247g) Aminocyclohexan wurde 4 h bei Raumtemperatur bis 120°C gerührt. Das Reaktionsprodukt wurde abfiltriert und 465 g Rohprodukt (klare, farblose Flüssigkeit) erhalten.
Der Umsatz, bestimmt mittels GC, betrug 97 %.

### Beispiel 3

16g eines Polyethylenimins (0,2 mol primäre und sekundäre Aminofunktionen, Lupasol® FG, BASF AG) und 1 mol (88 g) Ethylencarbonat wurde 3 h bei 60 -100°C gerührt. Das Mischung wurde heiß abfiltriert und 23 g Rohprodukt (gelblicher Feststoff) erhalten.

Eine Probe wurde entnommen, OH-Zahl bestimmt und mittels GPC analysiert. Der Umsatz der primären und sekundären Amine betrug über 90 %, wobei sich ein gegenüber dem Ausgangsprodukt um ca. 650 g/mol erhöhtes gewichtsmittleres Molgewicht M_{w} einstellte.

### Beispiel 4

Eine Mischung aus 0,5 mol (80g) des Reaktionsgemischs aus Beispiel 1, 1,0 mol (86g) Methyl-acrylat und 2g Novozym 435 (Lipase aus *Candida antartica* B) wurde 24 h bei 60°C gerührt. Das Enzym wurde abfiltriert, Methanol im Vakuum abrotiert und 86 g Rohprodukt (klare, farblose Flüssigkeit) erhalten.

Das Produkt ist in gängigen Polyether, -ester und urethanacrylaten löslich und ohne Eintrübungen zumischbar.

Eine Probe wurde entnommen, silyliert und mittels GC analysiert. Das Reaktionsgemisch aus Beispiel 1 war zu >97 % umgesetzt worden.

### Beispiel 5

Eine Mischung aus 18,7 g (0,1 mol) des Reaktionsgemischs aus Beispiel 2, 1,0 mol (86g) Methylacrylat und 2g Novozym 435 (Lipase aus *Candida antartica* B) wurde 24 h bei RT gerührt. Das Enzym wurde abfiltriert, Methanol im Vakuum abrotiert und 26 g Rohprodukt (klare, farblose Flüssigkeit) erhalten.

Eine Probe wurde entnommen, silyliert und mittels GC analysiert. Das Reaktionsgemisch aus Beispiel 2 war zu > 95 % umgesetzt worden.

### Beispiel 6

Eine Mischung aus 0,1.mol (OH-Äquivalent, 7 g) des Reaktionsgemischs aus Beispiel 3, 1,0 mol (86,1 g) Methylacrylat und 2,0 g Novozym 435 (Lipase aus *Candida antarti*ca B) wurde 24 h bei 60°C gerührt. Das Enzym wurde abfiltriert, Methanol im Vakuum abrotiert und 15 g Rohprodukt (gelblicher Feststoff) erhalten.

Eine Probe wurde entnommen, OH-Zahl bestimmt und mittels GPC analysiert. Die Alkoholfunktionalitäten sind zu > 95 % umgesetzt worden. Es ergab sich ein gegenüber dem Ausgangsprodukt um ca. 490 g/mol erhöhtes gewichtsmittleres Molgewicht M_{w}.

### Beispiel 7

Eine Mischung aus 5 mmol (746 mg) 2-Hydroxyethyl-N-[3'-(2"-hydroxyethyl-N'-propylcarba-moyl)]carbamat, 100 mmol (8,61 g) Methylacrylat, 1 g 5 Å Molsieb und 75 mg Novozym 435 (Lipase aus Candida antartica B) evtl. 5 ml Aceton wurde bei der angegebenen Temperatur gerührt. Das Enzym wurde abfiltriert, Methylacrylat abrotiert und 1,4 g Rohprodukt (klare, gelbliche Flüssigkeit) erhalten.

Eine Probe wurde entnommen, silyliert und mittels GC analysiert. Laut GC-Analyse setzte sich das Produkt wie folgt zusammen:

| Ansatz | Bedingungen | Umsatz [%]a | Monoacrylat [%] | Diacrylat [%] |
|---|---|---|---|---|
| 1 | 24h/40°C, ohne Aceton | 62 | 84 | 16 |
| 2 | 24h/60°C, ohne Aceton | 90 | 57 | 43 |
| 3 | 24h/60°C, ohne Molsieb, mit Aceton | 82 | 93 | 7 |
| 4 | 48h/50°C, ohne Aceton | 98 | <2 | 98 |

| | | | | |
|---|---|---|---|---|
| a Umsatz zu Mono- und Diacrylat insgesamt | | | | |

### Beispiel 8

Eine Mischung aus 1,0 mol (119,1 g) Hydroxypropylcarbamat (Isomerengemisch aus 2-Hydroxy-1-propylcarbamat und 3-Hydroxy-2-propylcarbamat), 10,0 mol (860 g) Methylacrylat, 172 mg 4-Methoxyphenol, 43 mg Phenothiazin, 300 g Molsieb (5 Å) und 9,0 g Novozym 435 (Lipase aus *Candida antartica* B) wurde 72 h bei 60 °C gerührt. Das Enzym und Molsieb
wurden abfiltriert, Methylacrylat im Vakuum abrotiert und 162 g Rohprodukt (klare, farblose Flüssigkeit) erhalten. Eine Probe wurde entnommen und mittels GC-MS analysiert. Das Hydroxypropylcarbamat war zu 96 % umgesetzt.

### Beispiel 9

Eine Mischung aus 5 mmol (525 mg) 2-Hydroxyethylcarbamat, 25 - 100 mmol Methylacrylat, 1,0 g Molsieb (5 A) und 30 mg Novozym 435 (Lipase aus *Candida antartica* B) wurde 8 h bei 60 °C in Abwesenheit eines Polymerisationsinhibitors gerührt. Eine Probe wurde entnommen und mittels GC analysiert. Die Umsätze des 2-Hydroxyethylcarbamat sind in der folgenden Tabelle beschrieben. Das Produkt fällt als farblose Kristalle an.

| Methylacrylat | Umsatz [%] mit Molsieb | Umsatz [%] ohne Molsieb |
|---|---|---|
| 50 mmol | 85 | 80 |
| 100 mmol | 99 | 89 |

### Beispiel 10

52,5 g Hydroxyethylcarbamat wurde mit einem Überschuß an Methylacrylat (129 g) mit 0,6 g Fascat® 4201 E-Coat (Dibutylzinnoxid, Elf Atochem) und Stabilisatoren (0,5 g MEHQ und 0,5 g BHT (Butylhydroxytoluol)) versetzt.

Zunächst wurde bei 80 °C für 2 Stunden gerührt, im Dünnschichtchromatogramm zeigte nur ein sehr geringer Umsatz. Es wurden dann 18 g Molsieb zugegeben. Die Reaktionstemperatur wurde zwischen 90 °C und 110 °C gehalten und für weitere 24 h gerührt. Das Molsieb wurde abfiltriert und dann das überschüssige Methylacrylat abdestilliert. Man erhielt eine klare, gelbe Flüssigkeit, die nach einem Tag anfing teilweise zu kristallisieren.

Die Ausbeute an Mischung betrug (laut NMR), ca. 80 %. Eine Probe der Mischung wurde entnommen, und per GC-MS analysiert. Es zeigten sich neben dem gewünschten Produkt (ca. 40 % der Mischung) eine Reihe an Nebenprodukten (z.B. Glykol und acrylierte Glycolderivate).

### Vergleichsbeispiel

Eine Mischung aus 3 mmol (1,49 g) 2-Hydroxyethyl-N-[3'-(2"-hydroxyethyl-N'-propylcarba-moyl)]carbamat, 60 mmol (5,16 g) Methylacrylat, 0,04 g p-Toluolsulfonsäure (TSS) und 500 ppm Hydrochinonmonomethylether zur Stabilisierung wurde bis zum Sieden unter Rückfluss erhitzt. Anschliessend wird ein Azeotrop von Methanol und Methylacrylat abdestilliert. Nach ca. 24 Stun-den Reaktion wird abgekühlt und das acrylierte Carbamat durch Vakuumdestillation in einer Ausbeute von 81 % erhalten.

| Bedingungen | Umsatz [%]a | Monoacrylat [%] | Diacrylat [%] |
|---|---|---|---|
| 24hNergleichsbeispiel mit p-TSS | 81 | 61 | 12 |

| | | | |
|---|---|---|---|
| a Umsatz zu Mono- und Diacrylat insgesamt | | | |

### Lackaufbau

Die Beschichtungsmasse aus Beispiel 7 (Ansatz 4) wurde mit 4 Gew.% Photoinitiator Irgacure® 500 der Firma Ciba Speciality Chemicals gemischt.

Die erhaltenen Mischung wurde auf einem Aufbau bestehend aus kationischer Tauchlackierung, Füller und einem Basislack (Brilliantschwarz) der Fa. BASF Coatings AG, Münster, appliziert. Der Basislack wurde 10 Minuten bei 80°C vorgetrocknet und fünfmal unter einer undotierten Quecksilberhochdrucklampe (Leistung 120 W/cm) mit einem Lampenabstand zum Substrat von 12 cm und einer Bandgeschwindigkeit von 5 m/min belichtet.

Die Schichtdicke nach der Belichtung betrug etwa 50 µm.

Die Pendeldämpfung wurde nach DIN 53157 bestimmt und ist ein Maß für die Härte der Beschichtung. Die Angabe erfolgt in Pendelschlägen für einen 50µm Film. Hohe Werte bedeuten dabei hohe Härte.

Die Erichsentiefung wurde nach DIN 53156 bestimmt und ist ein Maß für die Flexibilität und Elastizität. Die Angabe erfolgt in Millimeter (mm). Hohe Werte bedeuten hohe Flexibilität.

Die Haftung mit Gitterschnitt wurde gemäß der DIN 53151 ermittelt und in Noten angegeben. Kleine Werte bedeuten hohe Haftung.

**Tabelle 1**

| | Lack 1 Polyesteracrylat Laromer® PE55W ohne/mit 20 Gew.% Beschichtungsmasse aus Bsp. 7, Ansatz 4 | Lack 2 aminmodifiziertes Polyetheracrylat Laromer® PO 84F ohne/mit 20 Gew.% Beschichtungsmasse aus Bsp. 7, Ansatz 4 |
|---|---|---|
| Belichtung [mJ/cm2] | 1900 | 1900 |
| Schichtdicke [µm] | 55 | 60 |
| Pendelhärte [sec] | 32/40 | 45/50 |
| Erichsentiefung nach Härtung [mm] | 6,3/5,5 | 4,6/5,0 |
| Haftung mit Gitterschnitt/Tesaabriss | 5/4-5 | 5/4-5 |

**Tabelle 2 (Verwendung flüssiger Produkte als Reaktivverdünner)**

| | Lack 3 80% Epoxyacrylat 20 Gew.% Beschichtungsmasse aus Bsp. 4 | Lack 4 80% Epoxyacrylat 20 Gew.% Beschichtungsmasse aus Bsp. 7, Ansatz 4 | Lack 5 (Vergl.) 80% Epoxyacrylat 20 Gew.% Hexandioldiacrylat |
|---|---|---|---|
| Belichtung [mJ/cm2] | 1900 | 1900 | 1900 |
| Schichtdicke [µm] | 55 | 60 | 50 |
| Viskosität [Pas] | 30 | 70 | 18 |
| Pendelhärte [sec] | 150 | 170 | 175 |
| Erichsentiefung nach Härtung [mm] | 1,8 | 1,3 | 0,8 |
| Haftung mit Gitterschnitt/Tesaabriss | 5/4 | 5/4 | 5/5 |

Das verwendete Epoxyacrylat ist durch Umsetzung von Bisphenol-A-diglycidylether mit Acrylsäure erhältlich.

## Patentansprüche

1. Verfahren zur Herstellung urethangruppenhaltiger (Meth)acrylsäureester (F) durch
c) Umsetzung eines urethangruppenhaltigen Alkohols (C) mit (Meth)acrylsäure oder einem Ester von (Meth)acrylsaure mit einem gesättigten Alkohol (D) und
d) gegebenenfalls Aufreinigung des Reaktionsgemisches aus c), **dadurch gekennzeichnet, dass**
man die Umsetzung c) in Gegenwart eines Enzyms (E) durchführt.

2. Verfahren nach Anspruch 1, **dadurch gekennzeichnet, daß** es sich bei dem Enzym (E) um eine Lipase, Esterase oder Protease handelt.

3. Verfahren nach Anspruch 1 oder 2, **dadurch gekennzeichnet, daß** man in Stufe c) den Umsatz auf mindestens 90 % einstellt.

4. Verfahren nach einem der vorstehenden Ansprüche, **dadurch gekennzeichnet, daß** man die Umsetzung c) bei 20 bis 80 °C durchführt.

5. Verfahren nach einem der vorstehenden Ansprüche, **dadurch gekennzeichnet, daß** der urethangruppenhaltige Alkohol (C) erhältlich ist durch
a) Umsetzung eines Amins (A) mit einem Carbonat (B) und
b) gegebenenfalls Aufreinigung des aus a) erhältlichen Reaktionsgemisches.

6. Verfahren nach Anspruch 5, **dadurch gekennzeichnet, daß** der urethangruppenhaltige Alkohol (C) erhältlich ist durch eine Umsetzung gemäß, 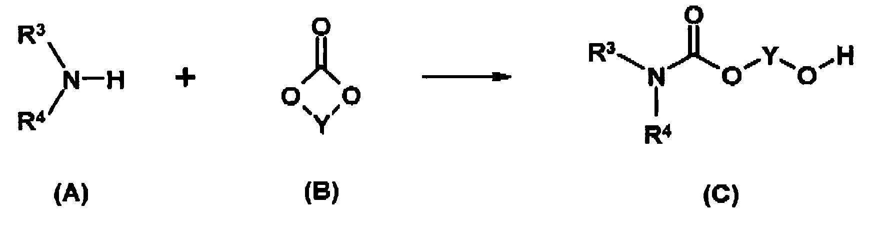 worin
R³, R⁴ unabhängig voneinander Wasserstoff. C₁ - C₁₈-Alkyl, gegebenenfalls durch ein oder mehrere Sauerstoff und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff- , Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, oder eine Gruppe der Formel -[Xₗ]ₖ-H,
Y C₂-C₂₀-Alkylen, C₅-C₁₂Cycloalkylen oder durch ein- oder mehrere Sauerstoffund/oder Schwefelatome und/oder ein oder mehrere substituierte oder unsubstituierte Iminogruppen und/oder durch eine oder mehrere Cycloalkyl-,
-(CO)-, -O(CO)O-,
-(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können,
k für eine Zahl von 1 bis 50 und
X₁ für i = 1 bis k voneinander ausgewählt sein kann aus der Gruppe -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂- CH₂-O-, -CH₂-CHVin-O-, -CHVin-CH₂-O-, -CH₂-CHPh-O- und -CHPh-CH₂-O-, worin Ph für Phenyl und Vin für Vinyl steht,
bedeuten.

7. Urethangruppenhaltiger (Meth)acrylsäureester, erhältlich durch
a) Umsetzung eines Polyethylenimins, eines hydrierten Polyacrylnitrils, eines geradkettigen, verzweigten oder dendritischen Polymers mit Aminofunktionen oder eines zumindest teilweise hydrolysierten Poly-N-Vinylformamids mit einem gewichtsmittleren Molekulargewicht M_{w} von 200 bis 1.000.000 mit einem Carbonat (B) bei einer Temperatur von 0 bis 120 °C,
b) gegebenenfalls Aufreinigung des aus a) erhältlichen Reaktionsgemisches,
c) Umsetzung des Reaktionsgemischs aus a) oder b) mit (Meth)acrylsäure oder einem Ester von (Meth)acrylsäure mit einem gesättigten Alkohol (D) in Gegenwart eines Enzyms (E) und
d) gegebenenfalls Aufreinigung des Reaktionsgemisches aus c).

8. Verfahren zur Beschichtung von Substraten, wobei man einen urethangruppenhaltigen (Meth)acrylsäureester (F) gemäß einem der Ansprüche 1 bis 6 herstellt,
und man wenigstens eine Beschichtungsmasse, enthaltend
- urethangruppenhaltigen (Meth)acrylsaureester (F)
- mindestens eine polymerisierbare Verbindung (G) mit mehreren copolymerisierbaren, ethylenisch ungesättigten Gruppen,
- gegebenenfalls Reaktivverdünner (H),
- gegebenenfalls Photoinitiator (I) sowie
- gegebenenfalls weitere lacktypische Additive (J)
auf das zu beschichtende Substrat in der gewünschten Stärke aufbringt,
die gegebenenfalls enthaltenen flüchtigen Bestandteile der Beschichtungsmasse, gegebenenfalls unter Erhitzen, entfernt und
anschließend härtet.

9. Verfahren zur Herstellung von Copolymerisaten, wobei man einen urethangruppenhaltigen (Meth)acrylsäureester (F) gemäß einem der Ansprüche 1 bis 6 herstellt,
man den so hergestellten urethangruppenhaltigen (Meth)acrylsaureester (F) mit Monomeren ausgewählt aus der Gruppe bestehend aus
C₁-C₂₀-Alkyl(meth)acrylaten, Vinylaromaten mit bis zu 20 C-Atomen, Vinylester von bis zu 20 C-Atornen enthaltenden Carbonsäuren, ethylenisch ungesättigten Nitrilen, Vinylethern von 1 bis 10 C-Atome enthaltenden Alkoholen und aliphatischen Kohlenwasserstoffen mit 2 bis 8 C-Atomen und 1 oder 2 Doppelbindungen
radikalisch oder ionisch copolymerisiert.

## Claims

1. A process for preparing (meth)acrylic esters (F) containing urethane groups by
c) reacting an alcohol (C) containing urethane groups with (meth)acrylic acid or an ester of (meth)acrylic acid with a saturated alcohol (D), and
d) if appropriate, working up the reaction mixture from c), which comprises
conducting the reaction c) in the presence of an enzyme (E).

2. The process according to claim 1, wherein the enzyme (E) is a lipase, esterase or protease.

3. The process according to claim 1 or 2, wherein the conversion in stage c) is set to at least 90%.

4. The process according to any of the preceding claims, wherein the reaction c) is conducted at from 20 to 80°C.

5. The process according to any of the preceding claims, wherein the alcohol (C) containing urethane groups is obtainable by
a) reacting an amine (A) with a carbonate (B), and
b) if appropriate, working up the reaction mixture obtainable from a).

6. The process according to claim 5, wherein the alcohol (C) containing urethane groups is obtainable by a reaction thus 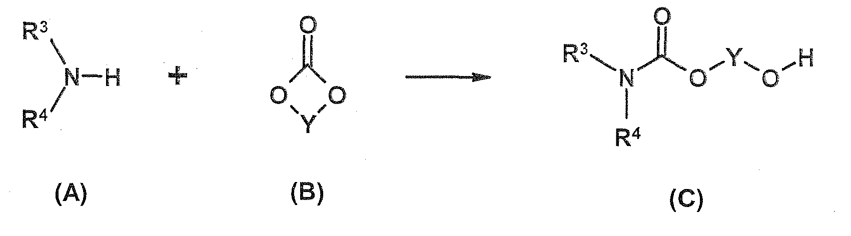 in which
R³, R⁴ independently are hydrogen, C₁-C₁₈ alkyl, C₂-C₁₈ alkyl optionally interrupted by one or more oxygen and/or sulfur atoms and/or by one or more substituted or unsubstituted imino groups, or are C₂-C₁₈ alkenyl, C₆-C₁₂ aryl, C₅-C₁₂ cycloalkyl or a five- to six-membered heterocycle containing oxygen, nitrogen and/or sulfur atoms, it being possible for each of the radicals stated to be substituted by aryl, alkyl, aryloxy, alkyloxy, heteroatoms and/or heterocycles, or are a group of the formula -[Xᵢ]ₖ-H,
Y is C₂-C₂₀ alkylene or C₅-C₁₂ cycloalkylene or is C₂-C₂₀ alkylene which is interrupted by one or more oxygen and/or sulfur atoms and/or by one or more substituted or unsubstituted imino groups and/or by one or more cycloalkyl, -(CO)-, -O(CO)O-, -(NH)(CO)O-, -O(CO)(NH)-, -O(CO)- or -(CO)O- groups, it being possible for each of the radicals stated to be substituted by aryl, alkyl, aryloxy, alkyloxy, heteroatoms and/or heterocycles,
k is a number from 1 to 50, and
xᵢ for i = 1 to k can be selected independently from the group consisting of -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂- CH₂-O-, -CH₂-CHVin-O-, -CHVin-CH₂-O-, -CH₂-CHPh-O-, and -CHPh-CH₂-O-, where Ph stands for phenyl and Vin stands for vinyl.

7. (Meth)acrylic esters containing urethane groups and obtainable by
a) reacting a polyethyleneimine, a hydrogenated polyacrylonitrile, a straight-chain, branched or dendritic polymer having amino functions or an at least partly hydrolyzed poly-N-vinylformamide having a weight-average molecular weight M_{w} of from 200 to 1 000 000 with a carbonate (B) at a temperature of from 0 to 120°C,
b) if appropriate, working up the reaction mixture obtainable from a),
c) reacting the reaction mixture from a) or b) with (meth)acrylic acid or with an ester of (meth)acrylic acid with a saturated alcohol (D) in the presence of an enzyme (E), and
d) if appropriate, working up the reaction mixture from c).

8. A method of coating substrates which involves preparing a (meth)acrylic ester (F) containing urethane groups, according to any one of claims 1 to 6,
and applying at least one coating composition comprising
- (meth)acrylic ester (F) containing urethane groups,
- at least one polymerizable compound (G) having two or more copolymerizable, ethylenically unsaturated groups,
- if appropriate, reactive diluent (H),
- if appropriate, photoinitiator (I), and
- if appropriate, further, typical coatings additives (J)
to the target substrate in the desired thickness,
removing any volatile constituents present in the coating composition, with heating if appropriate, and
carrying out subsequent curing.

9. A process for preparing copolymers which involves preparing a (meth)acrylic ester (F) containing urethane groups, according to any one of claims 1 to 6,
and subjecting the (meth)acrylic ester (F) containing urethane groups, prepared in this way, to free-radical or ionic copolymerization with monomers selected from the group consisting of
C₁-C₂₀ alkyl (meth)acrylates, vinylaromatics having up to 20 carbon atoms, vinyl esters of carboxylic acids comprising up to 20 carbon atoms, ethylenically unsaturated nitriles, vinyl ethers of alcohols comprising 1 to 10 carbon atoms, and aliphatic hydrocarbons having 2 to 8 carbon atoms and 1 or 2 double bonds.

## Revendications

1. Procédé de préparation d'esters d'acide (méth)acrylique contenant des groupes uréthanne (F) par :
c) réaction d'un alcool contenant des groupes uréthanne (C) avec de l'acide (méth)acrylique ou un ester d'acide (méth)acrylique avec un alcool saturé (D) et
d) éventuellement purification du mélange réactionnel de c), **caractérisé en ce que** on exécute la réaction c) en présence d'une enzyme (E).

2. Procédé selon la revendication 1, **caractérisé en ce qu'**il s'agit, pour l'enzyme (E), d'une lipase, d'une estérase ou d'une protéase.

3. Procédé selon la revendication 1 ou 2, **caractérisé en ce qu'**on règle à l'étape c) la conversion à au moins 90%.

4. Procédé selon l'une des revendications précédentes, **caractérisé en ce qu'**on effectue la réaction c) entre 20 et 80°C.

5. Procédé selon l'une des revendications précédentes, **caractérisé en ce que** l'alcool contenant des groupes uréthanne (C) peut s'obtenir par :
a) réaction d'une amine (A) avec un carbonate (B) et
b) éventuellement purification du mélange réactionnel obtenu de a).

6. Procédé selon la revendication 5, **caractérisé en ce que** l'alcool contenant des groupes uréthanne (C) peut s'obtenir par une réaction selon 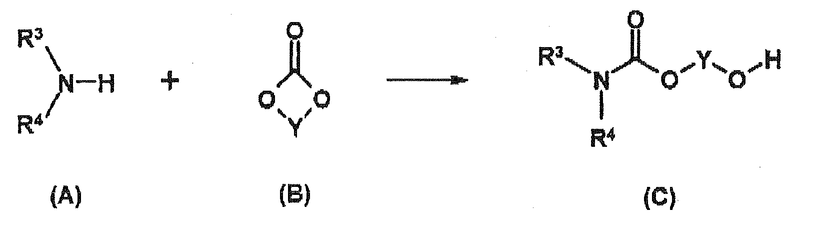 dans laquelle
R³, R⁴ signifient indépendamment l'un de l'autre un hydrogène, un alkyle en C₁-C₁₈, un alkyle en C₂-C₁₈ éventuellement interrompu par un ou plusieurs atomes d'oxygène et/ou de soufre et/ou un ou plusieurs groupes imino substitués ou non substitués, un alcényle en C₂-C₁₈, un aryle en C₆-C₁₂, un cycloalkyle en C₅-C₁₂ ou un hétérocycle de cinq à six membres présentant des atomes d'oxygène, d'azote et/ou de soufre, les radicaux mentionnés pouvant chaque fois être substitués par un aryle, un alkyle, un aryloxy, un alkyloxy, des hétéroatomes et/ou des hétérocycles, ou un groupe de formule - [Xi]ₖ-H,
Y signifie un alkylène en C₂-C₂₀, un cycloalkylène en C₅-C₁₂ ou un alkylène en C₂-C₂₀ interrompu par un ou plusieurs atomes d'oxygène et/ou atomes de soufre et/ou par un ou plusieurs groupes imino substitués ou non substitués et/ou par un ou plusieurs groupes cycloalkyle, -(CO)-, -O(CO)O-, -(NH)(CO)O-, -O(CO)(NH)-, -O(CO)- ou -(CO)O, les radicaux mentionnés pouvant être chaque fois substitués par un aryle, un alkyle, un aryloxy, un alkyloxy, des hétéroatomes et/ou des hétérocycles,
k représente un nombre de 1 à 50, et
Xᵢ peut être sélectionné pour i=1 à k dans le groupe des -CH₂-CH₂-O-, -CH₂-CH₂-N(H)-, -CH₂-CH₂-CH₂ -N (H) -, -CH₂-CH(NH₂)-, -CH₂-CH(NHCHO)-, -CH₂-CH (CH₃)-O-, -CH(CH₃)-CH₂-O-, -CH₂-C(CH₃)₂-O- ,-C(CH₃) ₂-CH₂-O-, -CH₂-CH₂-CH₂O-, -CH₂-CH₂-CH₂-CH₂-O-, -CH₂-CHVin-O-, -CHVin-CH₂-O, -CH₂-CHPh-O- et-CHPh-CH₂-O-, où Ph représente un phényle et Vin vinyle.

7. Ester d'acide (méth)acrylique contenant des groupes uréthanne, qu'on obtient par :
a) réaction d'une polyéthylènimine, d'un polyacrylonitrile hydrogéné, d'un polymère à chaîne droite, ramifié ou dendritique avec des fonctions amino ou d'un poly-N-vinylformamide au moins partiellement hydrolysé avec un poids moléculaire moyen en poids M_{w} de 200 à 1 000 000 avec un carbonate (B) à une température de 0 à 120°C,
b) éventuellement purification du mélange réactionnel obtenu en a),
c) réaction du mélange réactionnel de a) ou b) avec de l'acide (méth)acrylique ou un ester d'acide (méth)acrylique avec un alcool saturé (D) en présence d'une enzyme (E) et
d) éventuellement purification du mélange réactionnel de c).

8. Procédé de revêtement de substrats, dans lequel on prépare un ester d'acide (méth)acrylique (F) contenant des groupes uréthanne selon l'une des revendications 1 à 6,
et on applique au moins une matière de revêtement, contenant :
- un ester d'acide (méth)acrylique (F) contenant des groupes uréthanne,
- au moins un composé polymérisable (G) avec plusieurs groupes copolymérisables, éthyléniquement insaturés,
- éventuellement des diluants réactifs (H),
- éventuellement un photoinitiateur (I) ainsi que
- éventuellement d'autres additifs typiques aux peintures (J)
sur le substrat à revêtir à l'épaisseur souhaitée,
on élimine les constituants volatils éventuellement contenus de la matière de revêtement, éventuellement sous chauffage, et
on durcit ensuite.

9. Procédé de préparation de copolymères dans lequel on prépare un ester d'acide (méth)acrylique (F) contenant des groupes uréthanne selon l'une des revendications 1 à 6,
on copolymérise par voie radicalaire ou ionique l'ester d'acide (méth)acrylique (F) contenant des groupes uréthanne avec des monomères sélectionnés parmi le groupe composé
d'alkyl(méth)acrylates en C₁-C₂₀, de vinylaromatiques avec jusque 20 atomes de carbone, de vinylester d'acides carboxyliques contenant jusque 20 atomes de carbone, de nitriles éthyléniquement insaturés, de vinyléthers d'alcools contenant de 1 à 10 atomes de carbone et d'hydrocarbures aliphatiques avec 2 à 8 atomes de carbone et 1 ou 2 liaisons doubles.
